# EUROPEAN PATENT APPLICATION

(11) **EP 4 681 739 A1**
(43) Date of publication of application: **21.01.2026**
(21) Application number: 24189694.3
(22) Date of filing: 19.07.2024
(51) Int. Cl.: A61K 47/68, A61K 47/69, A61P 31/14, C12N 15/88, A61K 38/43

(54) **LIPID NANOPARTICLES FOR DIRECT DELIVERY**

(71) Applicant: PROVIREX Genome Editing Therapies GmbH, 22547 Hamburg (DE); Pantherna Therapeutics GmbH, 16761 Hennigsdorf (DE)
(72) Inventor: Fehring, Volker, 16761 Henningsdorf (DE); Keil, Oliver, 16761 Hennigsdorf (DE); Gkionis, Leonidas, 16761 Hennigsdorf (DE); Dunne, Charlotte, 16761 Hennigsdorf (DE); Wittner, Melanie, 22547 Hamburg (DE); Beschorner, Niklas, 22547 Hamburg (DE)
(74) Representative: Moré, Solveig Helga

(57) **Abstract**

The present invention relates to the field of lipid nanoparticles (LNP) and direct delivery of an active agent to a cell. It provides lipid nanoparticle constructs that are particularly useful for delivery of active agents, e.g., of RNA, preferably, mRNA, to cells. The lipid nanoparticle constructs comprise a specific neutral hydrophilic solid lipid nanpoparticle (SLP) and a targeting agent associated with the nanoparticle, e.g. a single domain antibody (sdAb), which may, for example target CD4. The lipid nanoparticle construct may comprise an active agent, and it may be used for targeting a cell such as a T cell. Pharmaceutical compositions comprising the nanoparticle constructs are also provided, in particular for use in targeting a cell. The invention also provides a composition or kit suitable for preparing the lipid nanoparticle construct of the invention.

## Description

The present invention relates to the field of lipid nanoparticles (LNP) and direct delivery of an active agent to a cell. It provides lipid nanoparticle constructs that are particularly useful for delivery of active agents, e.g., of RNA, preferably, mRNA, to cells. The lipid nanoparticle constructs comprise a specific hydrophilic lipid nanoparticle (LNP) and a targeting agent associated with the nanoparticle, e.g. a single domain antibody (sdAb), which may, for example target CD4. The lipid nanoparticle construct may comprise an active agent, and it may be used for targeting a cell such as a T cell. Methods of preparing a cell comprising an active agent and pharmaceutical compositions comprising the nanoparticle constructs are also provided, in particular for use in targeting a cell. The invention also provides a composition or kit suitable for preparing the lipid nanoparticle construct of the invention.

In recent years, nucleic acid drugs have shown a unique potential to convert previously "undruggable" targets into "druggable" ones, thus providing novel treatments. To overcome the challenges of poor *in vivo* stability and susceptibility to endogenous nucleases, LNPs are widely considered to be an ideal nucleic acid delivery system. However, the primary challenge associated with LNPs is their lack of targeting capabilities, leading to weak bioavailability of nucleic acid drugs and the potential for off-target effects, as well as *in vivo* toxicity. Therefore, developing strategies for enhancing LNPs' ability to target diverse organs is of great clinical significance. In particular, the predominant accumulation of LNPs in the liver poses a challenge to the successful development of nucleic acid therapeutics for extrahepatic diseases (Zhang et al 2024. iScience. 27(6),109804. 10.1016/j.isci.2024.109804).

LNPs are nanoparticles, i.e., typically spherical particles having an average diameter of at least 10 and less than 1000 nm, that are largely composed of lipids. Polymeric nanoparticles, self-emulsifying delivery systems, liposomes, microemulsions, micellar solutions and solid lipid nanoparticles have been widely tested as possible pharmaceutical drug delivery system. Liposomes are formed, typically, by a lipid bilayer encircling an aqueous core. Immunoliposomes can be functionalized and used for drug delivery (Eloy et al., 2017. Colloids and Surfaces B: Biointerfaces; 10.1016/j.colsurfb.2017.07.085). For example, liposomes conjugated to anti-CD4 have been used for targeting antiretroviral drugs to CD4 T cells Ramana et al., 2014. Eur. J. Biopharm, 10.1016/j.ejpb.2014.11.021).

LNPs as a drug delivery vehicle were first approved in 2018 for the siRNA drug Onpattro. Key components of LNPs are cationic or ionizable cationic lipids. The cationic charges help to bind an active agent having anionic charges, e.g., phosphate groups of RNA, in the LNP. Due to their pK value, ionizable lipids are positively charged under acidic conditions while remaining neutral at physiological pH 7.4. Cationic lipids maintain their cationic charge at said pH.

LNPs became more widely known in late 2020, as some COVID-19 vaccines that use RNA vaccine technology coat the fragile mRNA strands with PEGylated lipid nanoparticles as their delivery vehicle (including both the Moderna and the Pfizer-BioNTech COVID-19 vaccines, Pardi et al., 2018. Nature Reviews Drug Discovery 17(4). 261-279; https://doi.org/10.1038%2Fnrd.2017.243). LNPs used in the COVID-19 vaccines contain ionizable lipids whose positive charges bind to the negatively charged backbone of mRNA, pegylated lipids that help stabilize the particle, and phospholipids and cholesterol molecules that contribute to the particle's structure (Cross 2021. C & EN 99(8); https://cen.acs.org/pharmaceuticals/drug-delivery/Without-lipid-shells-mRNA-vaccines/99/i8; Wikipedia on Lipid nanoparticles).

Cargo molecules such as siRNA, mRNA, small molecule drugs or others can be embedded in the interior of LNPs, as desired. However, the pharmacological characteristics of LNPs depend on the LNP's size, structure and choice and ratio of components. One particularly important factor is the selection of lipids used for their production. Further, targeting-molecules such as antibodies, cell-targeting peptides, and/or other drug molecules can be bound to the exterior surface of the LNP.

US2023/0398082A1 teaches a lipid nanoparticle for extrahepatic delivery of mRNA that does not require a targeting agent. WO2023/152398A1 describes LNP suitable for local administration of an active agent such as RNA after local (e.g., intramuscular, subcutaneous, intravitreal, intrathecal, intratumoral, intracerebral and intradermal) administration. EP 3865122A1 discloses LNP for delivery of a therapeutic agent to pulmonary endothelium.

In vitro or in vivo targeting of LNP to leukocytes, in particular, to T cells, via surface antigens such as CD2, CD3, CD4, CD5 or CD7 has been suggested (WO2024/040194A1, WO2022/081694A1, WO2016/189532A1, Tombáct et al., 2021. Molecular therapy 29(11): 3293-3304, 10.1016/j.ymthe.2021.06.004; Shi et al. 2022. Journal of Controlled Release 345, 494-511, 10.1016/j.jconrel.2022.03.033).

As targeting of LNPs can be critical for different applications, there is a need in the art to provide further LNPs that are capable of addressing the desired selection of cells to deliver a desired cargo with high specificity, efficiency and low toxicity to desired cells. Designing LNPs requires the striking of a proper balance between the need for particle stability, long systemic circulation time, and the need for LNP destabilization inside the target cell to release the oligonucleotide cargo, which requires the proper selection of both the cationic and helper lipids. Customized design and empirical optimization is needed for specific applications (Cheng et al., 2016. Adv Drug Deliv Rev.99(Pt A):129-137, 10.1016/j.addr.2016.01.022).

In light of this, and to overcome at least some of the disadvantages associated, the inventors have addressed the problem of providing LNPs capable of specifically targeting cells via a targeting agent with a low level of unspecific transfection. The problem has been solved by the present invention as described herein, in particular, by the subject matter of the claims.

The invention provides a lipid nanoparticle construct comprising a hydrophilic lipid nanoparticle comprising:
a) a cationic lipid selected from a group consisting of β-(L-Arginyl)-L-2,3-diamino propionic acid-N-palmityl-N-oleyl-amide (PANTH02) and β-(L-Arginyl)-L-β-alanine-N-palmityl-N-oleyl-amide
b) 3β-Hydroxy-5-cholestene 3-hemisuccinate (cholesteryl hemisuccinate, CHEMS),
c) cholesterol, and
d) a PEGylated 1,2-diacyl-glycero-lipid selected from a group consisting of 1,2-diacyl-glycero-3-phosphoethanolamine-N-polyethylene glycol and 1,2-diacyl-glycero-3-O-polyethylenglycol, wherein the acyl groups are independently selected from a group consisting of myristoyl, palmitoyl, oleoyl and stearoyl,
and a targeting agent linked to the distal PEG-chain end of at least a part of the PEGylated 1,2-diacyl-glycero-lipid.

Optionally, the lipid nanoparticle further comprises a methoxy-terminated PEGylated 1,2-diacyl-glycero-lipid selected from a group consisting of 1,2-diacyl-glycero-3-phosphoethanolamine-N-polyethylene glycol and 1,2-diacyl-glycero-3-O-polyethylenglycol, wherein the acyl-groups are independently selected from a group comprising myristoyl, palmitoyl, oleoyl and stearoyl.

Accordingly, the term lipid nanoparticle construct is used herein forthe LNP associated with a targeting agent, while the LNP does not comprise a targeting agent.

In a preferred embodiment, the invention provides a lipid nanoparticle construct comprising a hydrophilic lipid nanoparticle comprising
a) β-(L-Arginyl)-L-2,3-diamino propionic acid-N-palmityl-N-oleyl-amide (PANTH02),
b) 3β-Hydroxy-5-cholestene 3-hemisuccinate (cholesteryl hemisuccinate, CHEMS),
c) cholesterol, and
d) 1,2-distearoyl-sn-glycero-3-phosphoethanolamine-N-polyethylene glycol (DSPE-PEG), preferably, DSPE-PEG2000
and a targeting agent linked to the distal PEG-chain end of at least a part of the PEGylated 1,2-diacyl-glycero-lipid. Optionally, the lipid nanoparticle further comprises a methoxy-terminated DSPE-PEG.

Accordingly, the term lipid nanoparticle construct is used herein forthe LNP associated with a targeting agent, while the LNP does not comprise a targeting agent.

The inventors have surprisingly found that, in contrast to other LNP constructs, the LNP constructs of the present invention, which comprise a targeting agent, are able to selectively target cells to which the targeting agent binds, and, accordingly, they can advantageously be used for delivery of an active agent to a desired cell. In particular, LNP constructs of the invention comprising a targeting agent to CD4 were able to efficiently transfect T cells, in particular, CD4 T cells, with an mRNA comprised in the LNP. Specific targeting reduces the risk of undesired effects in non-targeted cells, and of overall cytotoxicity. The LNP constructs of the present invention can thus be advantageously used for transfecting desired cells expressing an antigen recognized by the targeting agent in a cell mixture, e.g. in vitro. They can also be for use in in vivo direct delivery of an active agent to a desired cell expressing an antigen recognized by the targeting agent, e.g., in a human patient.

The LNPs of the present invention are hydrophilic colloids, i.e., they can be dispersed in an aqueous solvent such as water, physiological saline or a buffer. The LNP preferably does not comprise significant amounts of additional lipids, optionally, at most 10 mol% additional lipids, at most 5 mol% additional lipids, at most 1 mol% additional lipids or no additional lipids.

The LNPs of the present invention comprise a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide. Preferably, the cationic lipid is β-(L-Arginyl)-L-2,3-diamino propionic acid-N-palmityl-N-oleyl-amide (also designated PANTH02 herein). β-(L-Arginyl)-L-2,3-diamino propionic acid-N-palmityl-N-oleyl-amide can have Formula 1:

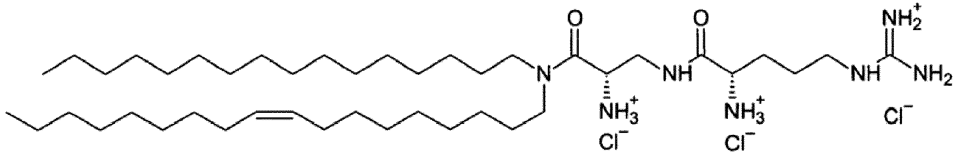

β-(L-Arginyl)-L-β-alanine-N-palmityl-N-oleyl-amide can have Formula 2:

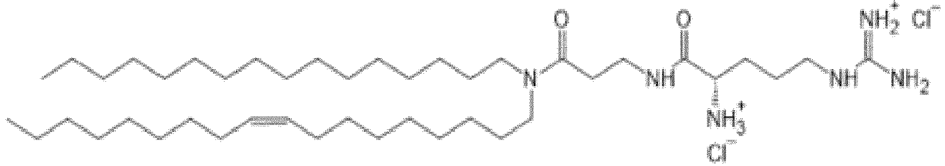

The lipid systems of the present invention are preferably strongly positively charged at lower pH values of pH 3-5 since the cationic lipids are highly positively charged at acidic pH values, but they also comprise anionic lipids that are not charged anymore due to the protonation of their carboxylic groups. These strongly positively charged lipid systems are capable of interacting electrostatically with negatively charged active agents such as RNA, to complex RNAs and to form LNPs incorporating the active agents accordingly. Once the LNPs of the present invention are formed and the pH of the dispersion medium is adjusted to a neutral pH value of 6.5-7.5, the cationic lipids of the LNPs are still positively charged due to the particularly high pKa-value of the arginine-guanidino-group, but at the same time the negative charges of the anionic lipids are regenerated due to the deprotonation of their carboxylic groups whereas the active agents, e.g., RNAs, are still trapped inside the LNP particles. The molar ratios between cationic lipids and anionic lipids in these amphoteric LNP systems are preferably chosen in a way that, at neutral pH values of pH 7.0-7.5, the number of cationic charges which are presented by the cationic lipids balances the number of the anionic charges which are presented by the anionic lipids. As a result, the LNP surface is decorated with about the same number of positive and negative charges. Therefore, the resulting LNP surface can be described as overall neutral.

Therefore, the LNPs are preferably neutral, i.e., the number of cationic and anionic charges in the LNP is preferably about the same at neutral pH.

CHEMS and cholesterol are helper lipids that may contribute to the LNP's stability and delivery efficiency. Cholesteryl hemisuccinate (CHEMS) is a weakly acidic amphiphilic lipid and thus can compensate the cationic charges of the cationic lipid at neutral pH of pH 7.0-7.5, The chemical structure of CHEMS is shown in Formula 3:

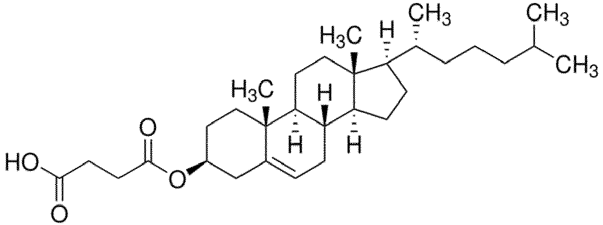

It may trigger low-pH-induced changes in LNP surface charge and destabilization that can facilitate endosomal release of active agents, e.g., oligonucleotides.

Cholesterol is a cone-shaped lipid and a natural component of the cell membrane. It may improve stability of LNPs and promote fusion with endosomal membranes upon uptake. It can be either synthetic or obtained from natural sources such as sheep wool. Cholesterol has Formula 4:

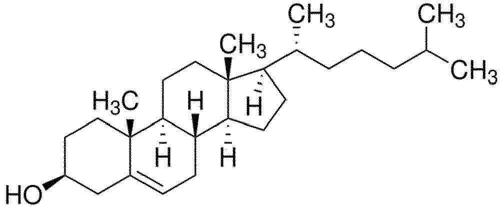

The LNPs of the present invention further comprise a PEGylated 1,2-diacyl-glycero-lipid, also designated PEGylated lipid herein. The PEGylated 1 ,2-diacyl-glycero-lipid is selected from the group consisting of 1,2-diacyl-glycero-3-phosphoethanolamine-N-polyethylene glycol and 1,2-diacyl-glycero-3-O-polyethylenglycol. 1,2-diacyl-glycero-3-phosphoethanolamine-N-polyethylene glycol is preferred. The acyl groups are independently selected from a group consisting of stearoyl, myristoyl, palmitoyl, and oleoyl. The 2-acyl-glycero-group can be either rac or sn configurated, preferably, *sn* configurated.

The lipid nanoparticle construct of the invention further comprises a targeting agent linked to the distal PEG-chain end of at least a part of the PEGylated 1,2-diacyl-glycero-lipid. The PEGylated 1,2-diacyl-glycero-lipid comprises a PEG chain that has a proximal end linked to the 1,2-diacyl-glycero-lipid and a distal end which, in at least a part of the molecules, optionally, in all cases, is linked to the targeting agent. In the PEGylated 1,2-diacyl-glycero-lipid linked to the targeting agent, preferably, the acyl groups are stearoyl groups. This allows for a particularly good anchoring of the targeting agent in the LNP.

In one embodiment, all PEGylated lipids are linked to targeting agent. This is however not required.

Therefore, optionally, the lipid nanoparticle further comprises a methoxy-terminated PEGylated 1,2-diacyl-glycero-lipid, i.e., a PEGylated 1,2-diacyl-glycero-lipid that is not linked to the targeting agent, but bears a methoxy-group at the distal end of the PEG chain. Preferably, all PEGylated lipid not linked to targeting agent is methoxy-terminated PEGylated lipid. The methoxy-terminated PEGylated 1,2-diacyl-glycero-lipid is selected from a group consisting of 1,2-diacyl-glycero-3-phosphoethanolamine-N-polyethylene glycol and 1,2-diacyl-glycero-3-O-polyethylenglycol, wherein the acyl-groups are independently selected from a group comprising myristoyl, palmitoyl, oleoyl and stearoyl. The 2-acyl-glycero-group can be either *rac* or *sn* configurated, preferably, sn configurated.

In a preferred embodiment, the PEGylated 1,2-diacyl-glycero-lipid linked to the targeting agent and the methoxy-terminated PEGylated 1,2-diacyl-glycero-lipid otherwise have the same chemical structure, e.g., they have the same acid groups, the same configuration and they are both either 1,2-diacyl-glycero-3-phosphoethanolamine-N-polyethylene glycol or 1,2-diacyl-glycero-3-O-polyethylen-glycol. However, this is not required. For example, it can be beneficial if at least a part, optionally all acyl groups of the methoxy-terminated PEGylated 1,2-diacyl-glycero-lipid are diffusible lipid-PEGs, i.e., less well anchored PEG-lipids. Without intending to be bound by the theory, LNP constructs comprising PEGylated lipids wherein the acyl groups are myristoyl, palmitoyl or oleyl can lose some of the PEGylated lipids over time after administration, which may make them easier to take up into target cells.

Preferred methoxy-terminated PEGylated 1,2-diacyl-glycero-lipid are:
- methoxy-terminated PEG-DSPE, mPEG-DSPE (Formula 5): 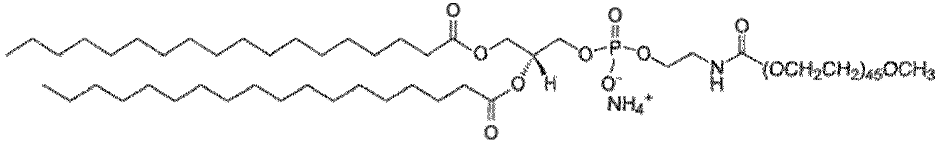
- mPEG-DSG (Formula 6):

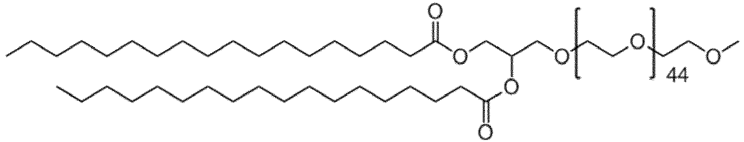

In a preferred embodiment, throughout the invention, the LNP comprises DSPE-PEG, at least as the targeting agent-linked PEGylated lipid, and optionally, also in the form of methoxy-terminated PEGylated lipid.

The polyethylene glycol (PEG) can be of different molecular weight, e.g., in the range between PEG750 and PEG5000, e.g., PEG 1000, preferably, between PEG1500 and PEG3000. Excellent results were obtained with PEG2000. For example, in a preferred embodiment, the targeting agent is linked to DSPE-PEG2000.

Throughout the invention, the molar ratio of the lipids in the lipid nanoparticle can be, e.g.,
from 20-40 mol% cationic lipid (e.g. β-(L-Arginyl)-L-2,3-diamino propionic acid-N-palmityl-N-oleyl-amide), preferably, 25-35 mol%, optionally, about 30 mol%,
from 39-59 mol% CHEMS, e.g., 49 mol%,
from 8.5-28.5 mol% cholesterol and, e.g., 18.5 mol%,
from 0.5-5 mol% PEGylated 1,2-diacyl-glycero-lipid (e.g., DSPE-PEG), preferably, about 2.5 mol%,
wherein the overall lipid content is 100%.

In this context, PEGylated 1,2-diacyl-glycero-lipid comprises both PEGylated 1,2-diacyl-glycero-lipid linked to targeting agent and methoxy-terminated PEGylated 1,2-diacyl-glycero-lipid, if present. The percentage of PEGylated 1,2-diacyl-glycero-lipid linked to targeting agent can be 20-100 mol%, e.g., 30-90 mol% or 50-80 mol% or 6-70 mol% of the total PEGylated 1,2-diacyl-glycero-lipid. The rest, to make up 100 mol% of PEGylated 1,2-diacyl-glycero-lipid, is substantially or, preferably, completely methoxy-terminated PEGylated 1,2-diacyl-glycero-lipid.

The molar ratio of cationic lipid (e.g., β-(L-Arginyl)-L-2,3-diamino propionic acid-N-palmityl-N-oleyl-amide) can be, e.g., 25-35 mol% or 28-32 mol%, preferably, 30 mol% of all lipids. The molar ratio of CHEMS can be, e.g., from 44-54 mol%, or 47-51 mol%, preferably, 49 mol% of all lipids. The molar ratio of cholesterol can be, e.g., 13.5-23.5 mol% or 17-20 mol%, preferably, 18.5 mol% of all lipids. The molar ratio of PEGylated lipid (e.g., DSPE-PEG) can be, e.g., 1-4 mol%, e.g., 1.5-3 mol%, or 2-3 mol%, preferably, 2.5 mol% of all lipids. Optionally, the molar ratio cationic lipid : CHEMS : cholesterol : PEGylated lipid is 25-35 : 44-54 : 13.5-23.5 : 1.5-3. In a preferred embodiment, the molar ratio β-(L-Arginyl)-L-2,3-diamino propionic acid-N-palmityl-N-oleyl-amide : CHEMS : cholesterol : DSPE-PEG is 25-35 : 44-54 : 13.5-23.5 : 1.5-3.

Preferably the molar ratio cationic lipid : CHEMS : cholesterol : PEGylated lipid (e.g., β-(L-Arginyl)-L-2,3-diamino propionic acid-N-palmityl-N-oleyl-amide : CHEMS : Cholesterol : DSPE-PEG) is 30:49:18.5:2.5.

Particle size of the LNPs may e.g. be determined by Dynamic Light Scattering using a Zetasizer Ultra (Malvern Panalytical Ltd, Malvern, UK). All measurements are preferably carried out in an aqueous buffer as dispersant, which is a 270 mM sucrose solution buffered with 10 mM TRIS at pH 7.5. Dynamic Light Scattering is, for example, described in more detail in Stetefeld et al., 2016. Biophys Rev 8, 409-427; and Thomas 1987. Journal of Colloid and Interface Science 117, 187-192. The particle size of the LNP construct of the invention according to the Dynamic Light Scattering measurements is typically below 200 nm, below 100 nm, preferably, within a range of about 30 nm to about 90 nm and most preferably within a range of about 50 nm to about 70 nm.

In a preferred embodiment, particle size is determined as "Intensity Mean Peak" which is calculated by the measurement and analysis software ZX Explorer (Malvern Panalytical Ltd, Malvern, UK).

The polydispersity index (PDI) of LPNs of the invention is the square of the light scattering polydispersity and thus a measure for the polydispersity of the size distribution. It is calculated together with the "Intensity Mean Peak" by the software ZX Explorer (Malvern Panalytical Ltd, Malvern, UK). The PDI can be, e.g., <0.2, preferably, < 0.15.

The zeta potential of the particles of the invention is determined by Laser Doppler Electrophoresis using a Zetasizer Ultra (Malvern Panalytical Ltd, Malvern, UK). All measurements are preferably carried out in an aqueous buffer as dispersant, which is a 270 mM sucrose solution buffered with 10 mM TRIS at pH 7.5. Laser Doppler Electrophoresis is, for example, described in more detail in Clogston, J. D. & Patri, A. K. in "Characterization of Nanoparticles Intended for Drug Delivery" 697, 63-70 (Humana Press, 2011 ); and Sze, A., Erickson, D., Ren, L. & Li, D. "Zeta-potential measurement using the Smoluchowski equation and the slope of the current-time relationship in electroosmotic flow", Journal of Colloid and Interface Science 261, 402-410 (2003 ). The zeta potential of the LNP constructs of the invention according to the Laser Doppler Electrophoresis measurements is in a range of about -15 to 7 mV and preferably in a range of about -10 to +5 mV, e.g., -6 to +2 mV or, most preferably, about -5 to 0 mV.

More specifically, i.e. in a more preferred embodiment, zeta potential is determined as "Zeta Mean Peaks". Both expressions are calculated by the measurement and analysis software ZX Explorer (Malvern Panalytical Ltd, Malvern, UK).

Preferably, the lipid nanoparticle construct of the invention has a
a) Z-Average hydrodynamic diameter of 50 to 90 nm;
b) PDI < 0.15 and/or
c) ζ-potential of -10 to 5 mV

Preferably, the lipid nanoparticle construct has at least a Z-Average hydrodynamic diameter of 50 to 90 nm and a PDI < 0.15. Optionally, it also has a ζ-potential of -10 to 5, preferably, -5 mV. The lipid nanoparticle can, e.g., also have a Z-Average hydrodynamic diameter of 50 to 70 nm and a PDI < 0.1, but the ζ-potential is about -11 to 3, preferably, -4 mV.

The LNP construct comprises a targeting agent. Throughout the application, the word "a" is to be understood as meaning "one or more". Accordingly, the LNP construct can also comprise a plurality, e.g., two, different targeting constructs, e.g., for targeting different cells or for targeting cells expressing two surface markers. Preferably, there is only one targeting agent.

The targeting agent may be, e.g., selected from the group consisting of an antibody, an antigen binding fragment of an antibody and a ligand to a cell-surface receptor and a fragment of said ligand capable of binding to said receptor. There are different antibody formats that may be used. Antibodies of IgG isotype are useful for targeting, because they can be routinely produced, e.g., in the form of monoclonal antibodies. Antibodies can be human, murine, rat, rabbit, guinea pig, goat, sheep, avian, e.g., chicken, chimeric (e.g., comprising human constant domains and non-human, typically, murine variable domains) and or humanized antibodies (comprising a human framework and non-human, typically, murine complementarity determining regions CDRs). As it is preferred to use a small targeting agent, which, e.g., simplifies production, in one embodiment, the antibodies are scFv. Antigen-binding domains of antibodies such as Fab fragments or Fab₂ fragments can also be used.

In a preferred embodiment, the antibody is a single domain antibody. A single domain antibody (sdAb), also called nanobody, is an antibody fragment consisting of a single monomeric variable antibody domain, which is able to selectively bind to a specific antigen. They represent an excellent alternative to conventional antibodies (e.g., IgG): With a molecular weight of only 12-15 kDa, single-domain antibodies are much smaller than common antibodies (150-160 kDa) which are composed of two heavy protein chains and two light chains, and even smaller than Fab fragments (~50 kDa, one light chain and half a heavy chain) and single-chain variable fragments (~25 kDa, two variable domains, one from a light and one from a heavy chain). In particular, the single domain antibodies of the invention can be derived from antibodies from camelids, such as llamas, camels, dromedaries or alpacas. Camelids have a unique subset of immunoglobulins consisting of heavy chain homodimers devoid of light chains. Their variable region (VHH) is the smallest antigen-binding fragment found in the antibody world, and as a single polypeptide chain it is especially suitable for protein engineering. Single domain antibodies, or "nanobodies", can be the recombinant minimal-sized, intact antigen-binding domains derived from the VHH region of these heavy-chain antibodies. Alternatively, single domain antibodies can be derived from antibodies of cartilaginous fish, such as shark, which have heavy chain antibodies from which single domain antibodies called VNAR fragments can be obtained.

Unlike monoclonal antibodies, single domain antibodies can be readily produced in large amounts in simple bacterial expression systems. Moreover, single domain antibodies are usually extremely stable, can bind antigens with affinities in the nanomolar range, and are smaller in size and thereby easier to manipulate genetically as compared with antibody fragments such as single chain variable fragments.

Also, due to the small size and compact folding, single domain antibodies show a high chemical stability, solubility and fast tissue penetration. Additionally, single domain antibodies can be easily converted into multivalent formats, such as bivalent, trivalent, tetravalent or other multi-valent, optionally, multi-specific or biparatopic constructs, e.g. addressing different epitopes on the same antigen. The single domain antibodies can also be part of such constructs, e.g., two or more single domain antibodies may be linked together and then used as a targeting agent. Multivalency can increase avidity.

Also, single domain antibodies have comparable antigen specificities and affinities to classical immunoglobulins. Due to their high homology with human antibody (VH) fragments, show only very low immunogenicity. Further, single domain antibodies can optionally be humanized to further reduce immunogenicity.

The targeting agent can alternatively be a ligand to a cell-surface receptor or a fragment of said ligand capable of binding to said receptor. The targeting agent may be a peptide, but that is not required. For example, the ligand can be a sugar, e.g., mannose, which can target LNPs to specific cells expressing lectins which bind the sugar. The ligand can also, e.g., be insulin, such that cells expressing an insulin-receptor are targeted. In another embodiment, the targeting agent is a T cell receptor capable of binding to MHCI or MHCII in complex with a specific peptide epitope, which in this case is the cell-surface receptor. The peptide can e.g. be a peptide from a tumor. For example, the peptide may only be presented on the MHC in tumor cells bearing certain mutations. Accordingly, the TCR can target LNP to tumor cells. Alternatively, the peptide can be a peptide derived from an infective agent, e.g., a virus, such that the LNP is targeted to an infected cell. In one embodiment, the ligand is a cytokine or chemokine, and the receptor is the respective receptor.

In a preferred embodiment, the targeting agent is capable of binding to a surface antigen of a hematopoietic cell, e.g., an antibody such as a single domain antibody. Hematopoietic cells can be, e.g., hematopoietic stem cells, which for example express CD34. For example, the targeting agent can be a single domain antibody to CD34, e.g., for use in targeting to hematopoietic stem cells. To target hematopoietic cells which can be myeloid cells, e.g., macrophages, monocytes and/or dendritic cells the targeting reagent could e.g. be a single domain antibody to CD45. Hematopoietic cells can also be lymphoid cells, e.g., B cells, T cells, NK calls or NK/T cells. For example, surface antigens of NK/T cells are CD56 and CD16. Preferably, the targeting agent is capable of binding to a surface antigen of a T cell. The T cell can be a CD4 T cell, a CD8 T cell or a gamma delta T cell. Preferably, it is a CD4 T cell. In this case, the targeting agent can e.g. be capable of binding to CD4. The surface antigen of a T cell can for example be selected from CD4, CD8 (CD8a or CD8b), CD3, CD5, CD7, CD19 or CD28. Regulatory T cells can be targeted via CD25. Specific activation or differentiation stages such as naive cells, effector cells or memory cells can e.g., be targeted via the surface antigens PECA;1, CD103, CCR7, CD127, CD62L, CCR5, TBX21, GZMA, CD69, ITGAE, CD45RA, CD45RO CD38 or CTLA4 (Estipona et al, 2020, A guide to T cell markers, https://www.biocompare.com/Editorial-Articles/569888-A-Guide-to-T-Cell-Markers/). Targeting agents, e.g., antibodies or antibody fragments capable of binding to any of these antigens can thus be used for targeting the LNPs of the invention to the cells expressing the respective antigen.

In a particularly preferred embodiment, the targeting agent is an antibody or an antigen binding fragment thereof capable of binding to CD4, optionally, to human CD4. For example, the inventors have shown that a targeting agent that is a single domain antibody to human CD4, e.g., the specific nanobody used in the example below, shows good results in targeting the LNP of the invention to CD4 T cells.

If the LNP construct of the invention is to be used for targeting cells of a specific species, the targeting agent targets an antigen expressed in cells of that species. If the target cells are human cells, the targeting agent preferably targets a human antigen. If the target cells are mouse cells, the targeting agent preferably targets a mouse antigen. Of course, targeting agents can also be cross-reactive.

Throughout the invention, the targeting agent is linked to the PEG chain of the PEGylated 1,2-diacyl-glycero-lipid, in particular, to the distal end of the PEG-chain of at least part of the PEGylated 1,2-diacyl-glycero-lipid, e.g., DSPE-PEG. "Linked with or "associated with" in this context preferably means coupled or associated via a covalent linkage. However, this is not required, as an association via a noncovalent linkage, e.g., via Biotin-Streptavidin or Biotin-Avidin or another non-covalent linkage, preferably, with at least the same affinity as biotin-streptavidin is also possible. Non-covalent linkage may simplify preparation of kits that can be targeted to different cells via different targeting agents.

LNP can be produced as wrapsomes, which comprise a core composed of a cationic lipid, PEGylated neutral lipid and the active agent, e.g., an RNA, enveloped in a PEGylated neutral lipid and helper lipids on the surface. A process for preparation of wrapsomes is e.g., disclosed in Kuboyama et al., 2019. ACS Med. Chem. Lett. 10, 749-753; 10.1021/acsmedchemlett.8b00652. However, in the context of the present invention, the LNP preferably is obtainable from at least components a) - d), by a single step process. This single step process includes mixing the lipids (which can be done in a premixing step), typically also, mixing the active agent with the lipids and formation of LNP, preferably, using a microfluidic mixer.

The targeting agent can, at that stage, already be linked to the PEGylated lipid. However, preferably, the targeting agent is coupled to the LNP after formation of the LNP, i.e., there may be a separate coupling step introducing the targeting agent to form an LNP construct.

To that end, the PEGylated lipid can be activated to allow for conjugation of the targeting agent.

Conjugation can be by incorporation of activated PEGylated lipid in a LNP of the invention, which is then reacted with the targeting agent to form a conjugate. In this embodiment, the LNP construct of the invention does not have to comprise methoxy-terminated PEGylated lipid, because the activated PEGylated lipid already allows for formation of the LNP of the invention. Methoxy-terminated PEGylated lipid may still be present in addition to the activated PEGylated lipid, as long as there is, on the final LNP construct, sufficient targeting agent linked to allow for efficient targeting.
- In one embodiment, conjugation can be by Michael addition, e.g., by a thio-Michael addition, the PEGylated lipid can be linked to a sulfhydryl group (e.g., in a cysteine). This can be particularly advantageous if the targeting agent only comprises one cysteine, so that there is a directed coupling of the targeting agent in a specific configuration. The Michael-acceptor typically is a maleimide, in particular, maleimide-modified PEGylated lipid.
   For example, pre-reduced targeting agent can be mixed with maleimide-surface bearing LNPs, i.e., LNPs comprising maleimide-modified PEGylated 1,2 diacyl-glycero-lipid. The invention thus provides a lipid nanoparticle construct, obtainable from coupling reduced targeting agent to maleimide-modified PEGylated 1,2 diacyl-glycero-lipid, e.g., 1,2-distearoyl-sn-glycero-3-phosphoethanolamine-N-[maleimide(polyethylene glycol)], that has been incorporated in an LNP. The maleimide-modification preferably is at the distal end of the PEG-chain. The inventors have shown that this method leads to functional LNP capable of targeting the desired cells. DSPE-PEG-Maleimide is shown as Formula 7: 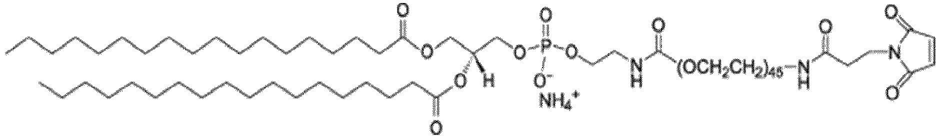
- Alternatively, coupling can also be via an alkyne azide cycloaddition reaction between a DBCO (dibenzocyclooctyne)-functionalised educt and an azide-functionalised educt.
   For instance, a DBCO-functionalised PEGylated lipid, typically DSPE-PEG can react with an azide group at the N-terminus of the targeting agent. The DBCO-modification preferably is at the distal end of the PEG-chain
   For example, the invention thus provides a lipid nanoparticle construct, obtainable from coupling azide-modified targeting agent to DBCO-functionalised PEGylated lipid, e.g., 1,2-distearoyl-sn-glycero-3-phosphoethanolamine-N-[dibenzocyclooctyne(polyethylene glycol)], that has been incorporated in an LNP. DSPE-PEG-DBCO is shown as Formula 8 : 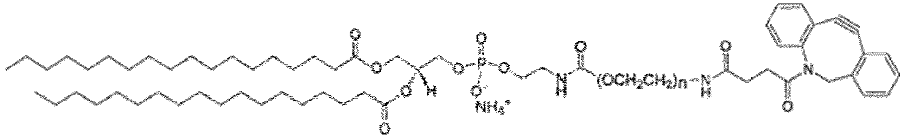
   In another embodiment, azide-functionalised PEGylated lipid, typically DSPE-PEG, can react with an DBCO group at the N-terminus of the targeting agent. The invention thus also provides a lipid nanoparticle construct, obtainable from coupling DBCO-modified targeting agent to 1,2-distearoyl-sn-glycero-3-phosphoethanolamine-N-[azido(polyethylene glycol)] that has been incorporated in an LNP. DSPE-PEG-Azide is shown as Formula 9:

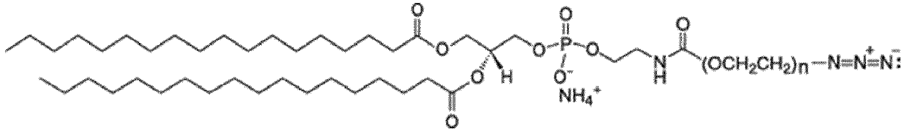

The targeting agent can also be inserted into the LNPs by a post-insertion process (post-PEGylation). In this case, the LNP already comprise mPEGylated lipids:
- In one embodiment, NHS (N-Hydroxysuccinimid)-ester-functionalised PEGylated lipids can react with the free amine group of the targeting agent's N-terminus The invention thus provides a lipid nanoparticle construct, wherein the targeting agent coupled to the PEGylated lipid, e.g., DSPE-PEG, is inserted into pre-formed LNPs. The LNP constructs are thus obtainable from incorporating PEGylated lipid, e.g., DSPE-PEG, that has been coupled to the targeting agent via reaction of NHS-ester-functionalised PEGylated lipid with the amine group at the N-terminus of the targeting agent. This can be particularly advantageous if the targeting agent has an amine group at the N-terminus of the protein, i.e., if it does not comprise lysine, because, in that case, there is a directed coupling of the targeting agent in a specific configuration. DSPE-PEG-NHS is shown as Formula 10: 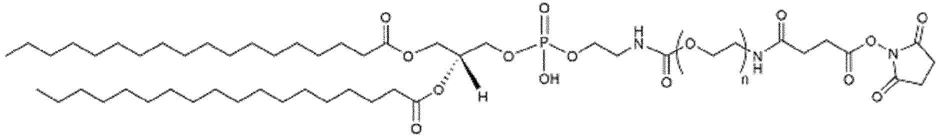
- In another embodiment, DBCO-functionalised PEGylated lipids can react with an azide group at the targeting agent's N-terminus in an alkyne azide cycloaddition. The invention thus provides a lipid nanoparticle construct, wherein the targeting agent coupled to PEGylated lipid, e.g., DSPE-PEG, is inserted into pre-formed LNPs. The LNP constructs are thus obtainable from incorporating PEGylated lipid, e.g., DSPE-PEG, that has been coupled to the targeting agent via reaction of DBCO-functionalised PEGylated lipid with an azide group at the N-terminus of the targeting agent.
- Alternatively, azide-functionalised PEGylated lipids can react with a DBCO-functionalized targeting agent, in particular, a DBCO group at the targeting agent's N-terminus. The invention thus provides a lipid nanoparticle construct, wherein the targeting agent coupled to PEGylated lipid, e.g., DSPE-PEG, is inserted into pre-formed LNPs. The LNP constructs are thus obtainable from incorporating PEGylated lipid, e.g., DSPE-PEG, that has been coupled to the targeting agent via reaction of azide-functionalised PEGylated DSPE with an DBCO group at the N-terminus of the targeting agent. DSPE-PEG-Azide is shown as Formula 11:

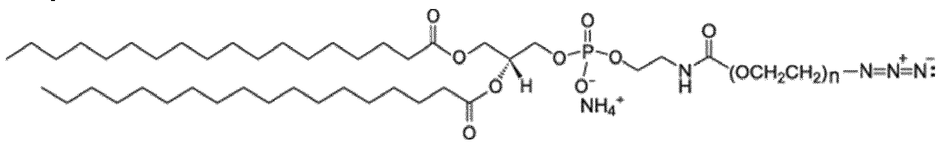

In such embodiments comprising post-PEGylation, PEGylated micelles bearing the targeting agent are mixed with preformed LNPs for insertion.

The modification of the targeting agent with a "clickable"-group can be performed e.g., by reacting the N-terminal amino-group of a targeting peptide sequence with either one of the following reagents, allowing for copper-free, strain promoted azide alkyne cycloaddition (SPAAC):
- Sulfo-DBCO-PEG4-maleimide (Formula 12) 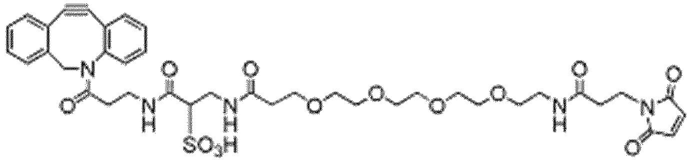
- 1-isothiocanato-PEG3-azide (Formula 13) 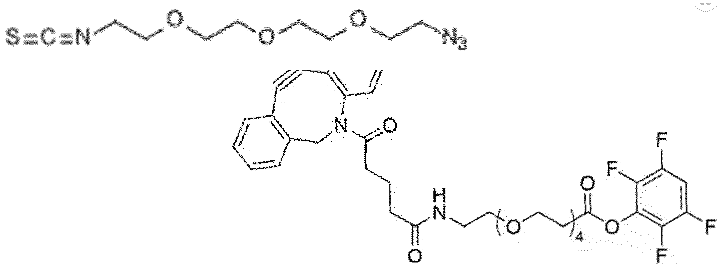
- DBCO-PEG4-TFP ester (Formula 14)

In another embodiment, the targeting agent is not covalently coupled to the PEGylated lipid, but non-covalently coupled. To this end, the LNP can comprise biotinylated PEGylated lipid, e.g., DSPE-PEG-biotin, wherein the biotin is linked to the distal end of the PEG chain, such as shown in Formula 15:

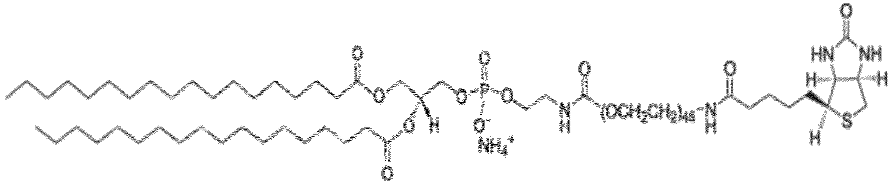

If biotin-modified PEGylated lipid is used, it is typically included in the LNP directly, in particular, in the one-step method of producing the LNP. In this case, the LNP construct can later be formed by adding streptavidin or avidin-linked targeting agent.

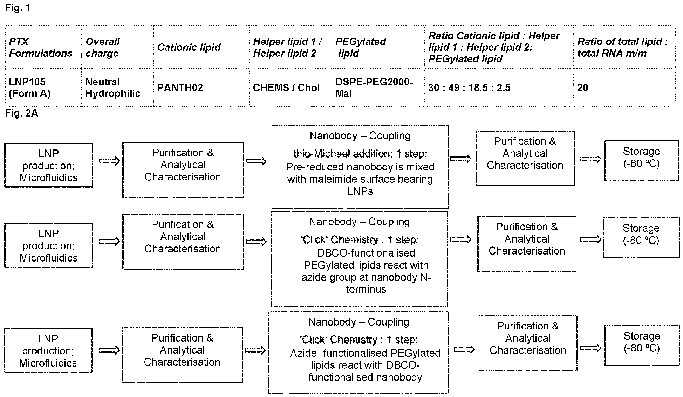

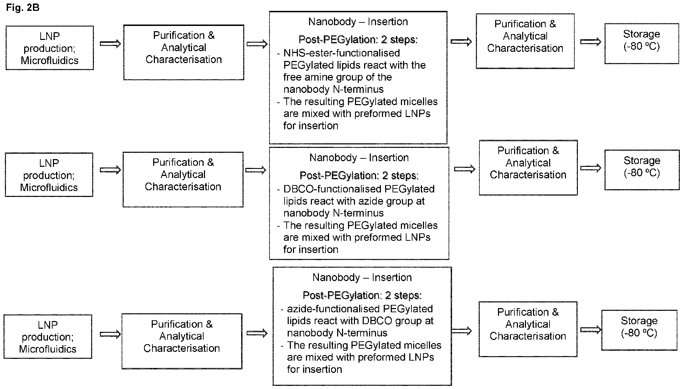

The different options for producing an LNP construct with targeting agent associated are further exemplified in the examples below or in Fig. 2 using a single domain antibody (nanobody). However, alternative targeting agents instead of single domain antibodies can also be used under the described conditions. Typically, the targeting agent in this context is a peptide (peptide is used synonymously with protein herein). The skilled person will be aware of corresponding methods of coupling non-peptide targeting agents.

The LNP construct typically comprises an active agent. In particular, the active agent is comprised in the LNP. Preferably, there is one active agent, but a combination of active agents can also be loaded in an LNP, e.g., two, three or four active agents. Preferably, active agents are negatively charged, in particular under conditions of loading the LNP, typically, at pH 3-5. Alternatively, the active agent can be lipophilic, e.g., a lipophilic dye. The active agent can be, e.g., an RNA, a DNA, a peptide, e.g., an enzyme, a drug, or a dye. Of course, more than one active agent can be comprised, e.g., a nucleic acid and a dye.

The active agent may be a therapeutically effective agent or the precursor of an agent having a therapeutic effect (i.e., a prodrug), e.g., an ester thereof. It may also be an agent that can be used for diagnosis, i.e., a diagnostically active agent, e.g., a dye. Preferably, to simplify loading, the active agent has at least one negative charge at a pH at which the cationic lipid has a positive charge. Alternatively, the active agent can be lipophilic, which also allows for loading into the LNP, e.g., a lipophilic dye.

In a preferred embodiment, the active agent is a nucleic acid, e.g., single stranded DNA, single stranded RNA, double stranded RNA or double stranded DNA. Preferably, it is RNA. Nucleic acids are negatively charged and therefore can advantageously be complexed with the cationic lipid. The nucleic acid may be, e.g., a plasmid DNA, a minicircle, a ssDNA, an oligodesoxynucleotide such as an antisense oligonucleotide, an aptamer, a spiegelmer, an siRNA, an asRNA, an miRNA, an sgRNA, an sa (selfamplifying) RNA, or in a preferred embodiment, mRNA, or a combination thereof. The nucleic acid may comprise a transposon, e.g., Sleeping Beauty, optionally, wherein the LNP further comprises a transposase in protein or nucleic acid, e.g., mRNA, form.

The nucleic acid, preferably, the mRNA, can encode a therapeutic gene. For example, it can encode an enzyme, e.g., a recombinase, i.e., an enzyme capable of recombining genomic DNA, such as site-specific recombinase, e.g., cre or a cre-derived enzyme, a zinc-finger nuclease, a TALEN or Cas (CRISPR associated endonuclease protein). If the nucleic acid encodes a Cas -encoding mRNA, the LNP further preferably also comprises a guide RNA, typically, a sgRNA.

The nucleic acid alternatively encodes a peptide capable of affecting an intracellular signaling pathway, e.g., a transcription factor or an enhancer. It can also encode a peptide capable of complementing an enzyme deficiency in a cell, e.g., an enzyme deficiency due to a hereditary or acquired disease.

In one embodiment, the nucleic acid, e.g., an mRNA, encodes a tumor suppressor gene, which can be helpful in treatment of cancer, in particular, in a cancer deficient in said tumor suppressor gene. The nucleic acid, e.g., the mRNA, can also encode a TCR or a CAR, which can harness the host cell, e.g., a T cells, such as a CD4 or C8 T cell, a gamma delta T cell or an NK or NKT cell to kill a cancer cell recognized by the TCR or CAR. If the nucleic acid is mRNA, transient expression may reduce the risk of negative side effects, e.g., if the antigen recognized by the TCR or CAR is an antigen also expressed by non-tumor cells.

In one embodiment, the nucleic acid, e.g., an mRNA, encodes an antigen to which an immune response is to be induced, e.g., a viral capsid protein such as a coronavirus protein or an influenza protein. This can be useful in the context of vaccination.

The nucleic acid can alternatively or additionally encode for a fluorescent protein, e.g., GFP, which can be helpful for testing transfection of cells, but it can also be used for diagnostic or analytical purposes. If the nucleic acid is DNA, the fluorescent protein can be encoded under the control of an inducible regulatory element, e.g., an inducible promotor, so that it can be tested if said regulatory element is activated in a cell. The nucleic acid can alternatively or additionally encode for an enzyme, e.g. luciferase, which can be helpful for in vivo distribution analysis.

Generally, if the nucleic acid is DNA, the nucleic acid can encode the therapeutic or diagnostic gene operably linked to an inducible regulatory element such as a promotor or a cell-specific promotor. This can be used to further increase specificity of expression in a desired target cell.

In a preferred embodiment, the active agent is a nucleic acid, preferably, an mRNA encoding a recombinase which is capable of excising a retroviral provirus from genomic DNA, e.g., as disclosed in WO2016/034553A1, such as Brec1 having SEQ ID NO: 11 of WO2016/034553A1, which is capable of excising HIV provirus from genomic DNA. The mRNA may also encode a recombinase capable of excising a HTLV-1 provirus as disclosed in PCT/EP2024/055000. Optionally, the recombinase may be encoded linked to a nuclear localization sequence. The targeting agent targets hosts cells of said provirus, e.g., T cells, in particular, CD4 T cells. Such LNPs may be for use in treatment of HIV infection or HTLV-infection, respectively. In a preferred embodiment, in the lipid nanoparticle construct of the invention, the targeting agent is a single domain antibody to human CD4 and the lipid nanoparticle comprises an mRNA encoding a recombinase, optionally, Brec1 or HTLV-Rec.

If the nucleic acid is RNA, it can be RNA modified to affect its stability and/or expression. Typically, the RNA is modified to stabilize it. mRNA is a nucleic acid molecule typically comprising 5'-> 3' direction, a Cap structure, a 5' untranslated region (5' UTR), a coding sequence typically starting with an AUG codon attached to a coding sequence (CDS) terminating with a stop codon, a 3' untranslated region (3' UTR) and a poly-A-tail. Preferably, the mRNA is made of ribonucleotides, e.g., L-ribonucleotides, D-ribonucleotides or a mixture thereof. RNA modifications which can be employed are, e.g., pseudouridine, 5-methoxy-uridine, 5-methylcytosine and preferably N1-methyl-pseudouridine. For example, an mRNA can comprise a Cap1 (3'OMe) structure, namely, m7(3'OMe)Gppp(2'OMe), and N1-Me-pseudouridine instead of uridine. Modifications can reduce immunogenicity of RNA and/or increase stability.

As shown by prior art, LNPs can also efficiently be used for delivery of inhibitory RNA, e.g., as in Onpattro.

The length of the nucleic acid, e.g., the RNA can be 15-20,000 nucleotides, preferably 20-2,000 nucleotides, 500-1,500 nucleotides, 700-1,400 nucleotides. It can e.g. have 200-700 nucleotides, 700-1,000 nucleotides, 1000-1,300 nucleotides or 1,300-1,500 nucleotides.

Nucleic acids, e.g., RNA, can be used at a lipid to nucleic acid mass ratio which is from 5-40, e.g., 10-35, 15-30 or preferably about 20 to about 28.

The active agent can also be a peptide, preferably comprising 50-1,000 aa, e.g., 80-800 aa. The pl of the peptide preferably is <5, e.g., <4. The peptide may be a therapeutic peptide. For example, it can be an enzyme, e.g., a recombinase, or an enzyme capable of recombining genomic DNA such as site-specific recombinase, e.g., cre or a cre-derived enzyme, a zinc-finger nuclease, a TALEN or Cas (CRISPR associated endonuclease protein). If the peptide is a Cas, the LNP further preferably also comprises a guide RNA, typically, a sgRNA.

The peptide alternatively is a peptide capable of affecting an intracellular signaling pathway, e.g., a transcription factor or an enhancer. It can also be a peptide capable of complementing an enzyme deficiency in a cell, e.g., an enzyme deficiency due to a hereditary or acquired disease.

In one embodiment, the peptide is an antigen to which an immune response is to be induced, e. g., a viral capsid protein such as a coronavirus protein or an influenza protein. This can be useful in the context of vaccination.

The peptide can alternatively or additionally be a fluorescent protein, e.g., GFP, which can be helpful for testing transfection of cells, but it can also be used for diagnostic or analytical purposes.

In a preferred embodiment, the active agent is a recombinase which is capable of excising a retroviral provirus from genomic DNA, e.g., as disclosed in WO2016/034553A1, preferably, Brec1 having SEQ ID NO: 11 of WO2016/034553A1, which is capable of excising HIV provirus from genomic DNA. The peptide may also be a recombinase capable of excising a HTLV-1 provirus as disclosed in PCT/EP2024/055000.

A drug as an active agent can e.g., be a small molecule, i.e., an organic molecule having at most 800 Da) that has a therapeutic or diagnostic effect. Suitable drugs are, e.g., a cytotoxic agent, an antiproliferative agent, an anti-inflammatory agent, an anti-infective agent, or an antibiotic agent.

Dyes can be used for diagnosis or for testing if specific cells are targeted by the LNP construct of the invention. Dyes - or RNA encoding a fluorescent protein can advantageously be used in combination with other active agents, e.g., if cells are in vitro contacted with the LNP constructs of the invention and then sorted according to the presence of the dye or fluorescent protein, optionally, before administration to a patient.

The invention also provides a composition comprising an LNP of the invention. The composition comprises a dispersion medium, in particular, the lipid nanoparticles may be dispersed in the dispersion medium. The medium forms a continuous phase. Preferably, it is an aqueous medium, e.g., water, a saline (e.g., physiological saline) or a buffer. The dispersion medium should be pharmaceutically acceptable if used for administration to a patient. It may, e.g., comprise a pharmaceutically acceptable aqueous buffer system. The medium may have a physiological pH (e.g., 7.0-7.6, preferably, 7.2-7.4) and isoosmolar strength (e.g., 250-330 mOsmol/kg). It may comprise a cryoprotectant, e.g., sucrose, glucose or trehalose or a mixture thereof, preferably, sucrose. It may e.g., be a 10 mM phosphate or Tris/HCl buffer of pH 7.0-7.6, preferably, pH 7.4, preferably, Tris/HCl, comprising 270 mM sucrose.

The composition may be liquid. It may alternatively be frozen, e.g., stored at -20 to -80°C. In this case, it preferably further comprises a cryoprotectant. It is thawed before use.

Disclosed is a method of preparing a cell comprising an active agent, comprising contacting said cell with the lipid nanoparticle construct of the invention comprising an active agent, wherein, optionally, the active agent is an mRNA encoding a therapeutic protein, and the cell expresses the therapeutic protein. The invention provides a method for preparing said cell, e.g., a transfected cell in vivo or in vitro, optionally, ex vivo.

The lipid nanoparticle construct of the invention can be used for targeting a cell such as a T cell in vitro, preferably, a CD4 or a CD8 T cell, most preferably, a CD4 T cell. The cell that is targeted by the LNP, typically, via the targeting agent, depends on the targeting agent. The cell can for example be a hematopoietic cell, e.g., a hematopoietic stem cell (which can e.g., be targeted with an antibody to CD34, such as a single domain antibody). It can be a myeloid cell (which can e.g., be targeted with an antibody to CD45, such as a single domain antibody) such as a dendritic cell, a monocyte or a macrophage. It can also be a lymphoid cell, e.g., a B cell, a T cell, e.g., an alpha beta T cell such as a CD4 or a CD8 T cell, or a gamma delta T cell, an NK cell or an NKT cell. Preferably, the cell is a CD4 T cell, wherein, optionally, the targeting agent is a CD4 single domain antibody or another single domain antibody capable of targeting a CD4 T cell-specific surface molecule, preferably, a CD4 single domain antibody.

In one embodiment, the invention provides a pharmaceutical composition comprising the lipid nanoparticle construct of the invention and a pharmaceutically acceptable solvent The pharmaceutically acceptably composition can be for use in targeting a cell such as a T-cell, optionally, a CD4 T cell. If the active agent is a recombinase capable of exciding HIV provirus, e.g., Brec1 (in nucleic acid or protein form, preferably, in nucleic acid form), the nanoparticle construct is for use in treating HIV infection, preferably, for preventing AIDS. As provirus can be completely eliminated by this approach, HIV infection can optimally be healed, and, e.g., antiretroviral therapy discontinued upon treatment success. Similarly, using HTLV recombinase as an active agent (in nucleic acid or protein form), a pharmaceutical composition of the invention can be for use in treating HTLV infection.

The pharmaceutical composition may be formulated for in vitro administration to a cell, preferably, ex vivo, and later re-administration, e.g., to the subject from which the cell, or precursors of it, had previously been derived. In vitro administration comprises contacting the cell - or a cell mixture comprising the target cell, e.g., in the case of CD4 T cells, PBMC - with the LNP construct of the invention. Conditions are choses to enable transfer of active agent to the desired target cells in a specific manner. Specific in this context means that the proportion of the desired target cells expressing the selected molecule bound by the targeting agent to which the target agent is transferred is higher than the proportion of the non-target cells not expressing the selected molecule bound by the targeting agent to which the target agent is transferred. Preferably, said proportion is at least 20% higher, at least 25% higher, at least 30% higher, at least 40 % higher, at least 50% higher, at least 75% higher, or at least 80% or at least 90% higher.

The pharmaceutical composition may also be for direct delivery to a subject, in particular, to a patient, namely for in vivo administration to a subject. Administration may be, e.g., intravenous, intramuscular, intrapulmonary, intranasal, intrathecal, topical, e.g., subcutaneous, subdermal, intratumoral, or oral.

The selection may depend on the targeted cell population, for example, if T cells are targeted, intravenous administration is preferred.

The patient preferably is a human patient. However, it can also be a mouse, a rat, a rabbit, a guinea pig, a monkey, a pig, a cow, a sheep, a goat, a camel, a chicken or a turkey. A patient has a disease or a condition in need of treatment. Accordingly, the pharmaceutical composition can be administered for use in treatment of a disease. It can also be administered for use in prevention of a disease, e.g., in vaccination. Prevention can be understood to reduce the incidence or risk or the symptoms of a disease. Thus, the

In another embodiment, the invention provides a composition or kit suitable for preparing the lipid nanoparticle construct of the invention. Said composition or kit may comprise
a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide, preferably, β-(L-Arginyl)-L-2,3-diamino propionic acid-N-palmityl-N-oleyl-amide, and
b) CHEMS,
c) cholesterol, and
d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group comprising stearoyl, myristoyl, palmitoyl, and oleoyl, preferably, stearoyl,
   wherein said PEGylated 1,2-diacyl-glycero-lipid is selected from maleimide-modified PEGylated 1,2-diacyl-glycero-lipid, DBCO-modified PEGylated 1,2-diacyl-glycero-lipid, azide-modified PEGylated 1,2-diacyl-glycero-lipid, targeting agent linked to the distal PEG-chain end of PEGylated 1,2-diacyl-glycero-lipid, biotinylated PEGylated 1,2-diacyl-glycero-lipid and/or methoxy-terminated PEGylated 1,2-diacyl-glycero-lipid,
e) wherein, if the PEGylated 1,2-diacyl-glycero-lipid of d) consists of methoxy-terminated PEGylated 1,2-diacyl-glycero-lipid, the composition or kit further comprises maleimide-modified PEGylated 1,2-diacyl-glycero-lipid, DBCO-functionalised PEGylated 1,2-diacyl-glycero-lipid, azide-functionalised PEGylated 1,2-diacyl-glycero-lipidor targeting agent linked to the distal PEG-chain end of PEGylated 1,2-diacyl-glycero-lipid,
f) wherein, if no linked targeting agent is present, the kit further comprises the targeting agent in separate form and
g) optionally, active agent.

Said kit or composition can then be used by a skilled person to prepare the LNP construct of the invention comprising a custom targeting agent and/or custom active agent as desired. Methods and reagents for preparing the LNP construct as disclosed herein can be used. A kit of the invention may further comprise instructions for preparing the LNP construct, e.g., in paper form.

The following figures and examples are intended to illustrate the invention, but not to limit its scope. "About", when applied to a range, should be understood to refer to both numbers of the range. "About" means +/- 10 %. The disclosure of all literature cited herein is herein incorporated by reference.

### The invention also provides the following embodiments

1. A lipid nanoparticle construct comprising a hydrophilic lipid nanoparticle comprising
   a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide,
   b) 3β-Hydroxy-5-cholestene 3-hemisuccinate (CHEMS),
   c) cholesterol, and
   d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group consisting of stearoyl, myristoyl, palmitoyl, and oleoyl,
   and a targeting agent linked to the distal PEG-chain end of at least a part of the PEGylated 1,2-diacyl-glycero-lipid.
2. The lipid nanoparticle construct of embodiment 1, wherein the lipid nanoparticle further comprises a methoxy-terminated PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group consisting of stearoyl, myristoyl, palmitoyl, and oleoyl.
3. The lipid nanoparticle construct of any of the preceding embodiments, wherein the cationic lipid is β- (L-Arginyl)-L-2,3-diamino propionic acid-N-palmityl-N-oleyl-amide.
4. The lipid nanoparticle construct of any of embodiments 1-2, wherein the cationic lipid is β-(L-Arginyl)-L-β-alanine-N-palmityl-N-oleyl-amide.
5. The lipid nanoparticle construct of any of the preceding embodiments, wherein the PEGylated 1,2-diacyl-glycero-lipid has a *sn* configuration.
6. The lipid nanoparticle construct of any of embodiments 1-4, wherein the PEGylated 1,2-diacyl-glycero-lipid has a *rac* configuration.
7. The lipid nanoparticle construct of any of the preceding embodiments, comprising PEGylated 1,2-distearoyl-glycero-lipids, optionally, DSPE-PEG.
8. The lipid nanoparticle construct of any of the preceding embodiments, wherein all PEGylated 1,2-diacyl-glycero-lipids are PEGylated 1,2-distearoyl-glycero-lipids, optionally, DSPE-PEG.
9. The lipid nanoparticle construct of any of embodiments 1-7, comprising methoxy-terminated PEGylated 1,2-dimyristoyl-glycero-lipids, optionally, wherein 5-75 mol%, e.g., 30-50 mol % of the PEGylated 1,2-diacyl-glycero-lipids are methoxy-terminated 1,2-dimyristoyl-glycero-lipids.
10. The lipid nanoparticle construct of any of embodiments 1-7 or 9, comprising methoxy-terminated PEGylated 1,2-dipalmitoyl-glycero-lipids, optionally, wherein 5-75 mol%, e.g., 30-50 mol% of the PEGylated 1,2-diacyl-glycero-lipids are methoxy-terminated 1,2-dipalmitoyl-glycero-lipids.
11. The lipid nanoparticle construct of any of embodiments 1-7 or 9-10, comprising methoxy-terminated PEGylated 1,2-dioleoyl-glycero-lipids, optionally, wherein 5-75 mol%, e.g., 30-50 mol% of the PEGylated 1,2-diacyl-glycero-lipids are methoxy-terminated 1,2- dioleoyl-glycero-lipids.
12. The lipid nanoparticle construct of any of the preceding embodiments, wherein the PEGylated 1,2-diacyl-glycero-lipid is 1,2-diacyl-glycero-3-phosphoethanolamine-N-polyethylene glycol.
13. The lipid nanoparticle construct of any of the preceding embodiments, wherein the PEGylated 1,2-diacyl-glycero-lipid is 1,2-distearoyl-sn-glycero-3-phosphoethanolamine-N-polyethylene glycol (DSPE-PEG), optionally, DSPE-PEG2000.
14. The lipid nanoparticle construct of any of the preceding embodiments, wherein the PEGylated 1,2-diacyl-glycero-lipid is PEGylated 1,2-diacyl-glycero-3-O-polyethylenglycol, optionally, PEGylated 1,2-distearoyl-glycero-3-O-polyethylenglycol.
15. The lipid nanoparticle construct of any of embodiments 1-8 and 12-14, wherein the targeting agent is linked to the distal PEG-chain end of all PEGylated 1,2-diacyl-glycero-lipid.
16. The lipid nanoparticle construct of any of embodiments 9-11, wherein the targeting agent is linked to the distal PEG-chain end of less than 100% PEGylated 1,2-diacyl-glycero-lipid, optionally, of 25%-90% of PEGylated 1,2-diacyl-glycero-lipid.
17. The lipid nanoparticle construct of any of the preceding embodiments, wherein the molar ratio of the lipids in the lipid nanoparticle is
   from 20-40 mol% cationic lipid, preferably, β-(L-Arginyl)-L-2,3-diamino propionic acid-N-palmityl-N-oleyl-amide
   from 39-59 mol% CHEMS
   from 8.5-28.5 mol% cholesterol and
   from 0.5-5 mol% PEGylated 1,2-diacyl-glycero-lipid, preferably, DSPE-PEG,
   wherein the overall lipid content is 100%.
18. The lipid nanoparticle construct of any of the preceding embodiments, wherein the molar ratio cationic lipid : CHEMS : cholesterol : PEGylated 1,2-diacyl-glycero-lipid is 30:49:18.5:2.5.
19. The lipid nanoparticle construct of any of the preceding embodiments having a
   a) Z-Average hydrodynamic diameter of 50 to 70 nm;
   b) PDI < 0.1 and/or
   c) ζ-potential of -9 to 1, preferably, -4 mV,
   preferably, a), b) and c).
20. The lipid nanoparticle construct of any of the preceding embodiments, wherein the targeting agent is capable of binding to a surface antigen of a hematopoietic cell.
21. The lipid nanoparticle construct of any of the preceding embodiments, wherein the targeting agent is capable of binding to a surface antigen of a T cell, optionally, a CD4+ T cell.
22. The lipid nanoparticle construct of any of the preceding embodiments, wherein the targeting agent is selected from the group consisting of an antibody, an antigen binding fragment of an antibody and a ligand to a cell-surface receptor and a fragment of said ligand capable of binding to said receptor.
23. The lipid nanoparticle construct of any of the preceding embodiments, wherein the targeting agent is an antibody.
24. The lipid nanoparticle construct of any of the preceding embodiments, wherein the targeting agent is a single domain antibody.
25. The lipid nanoparticle construct of any embodiments 1-21, wherein the targeting agent is an antigen binding fragment of an antibody.
26. The lipid nanoparticle construct of any embodiments 1-21, wherein the targeting agent is a ligand to a cell-surface receptor and a fragment of said ligand capable of binding to said receptor.
27. The lipid nanoparticle construct of any of the preceding embodiments, wherein the targeting agent is an antibody or an antigen binding fragment thereof capable of binding to CD4, optionally, to human CD4.
28. The lipid nanoparticle construct of any of the preceding embodiments, wherein the lipid nanoparticle is obtainable from at least components a) to d) by a single step process, optionally, with a separate coupling step introducing the targeting agent.
29. The lipid nanoparticle construct of any of the preceding embodiments, obtainable from coupling reduced targeting agent to maleimide-modified PEGylated 1,2-diacyl-glycero-lipid (e.g., DSPE-PEG) that has been incorporated in an LNP.
30. The lipid nanoparticle construct of any of embodiments 1-28, obtainable from coupling azide-modified targeting agent to dibenzocyclooctyne (DBCO)-modified PEGylated 1,2-diacyl-glycero-lipid (e.g., DSPE-PEG) that has been incorporated in an LNP.
31. The lipid nanoparticle construct of any of embodiments 1-28, obtainable from coupling dibenzocyclooctyne (DBCO)-modified targeting agent to azide-modified PEGylated 1,2-diacyl-glycero-lipid (e.g., DSPE-PEG) that has been incorporated in an LNP.
32. The lipid nanoparticle construct of any of embodiments 1-7 or 9-28, wherein the targeting agent coupled to PEGylated 1,2-diacyl-glycero-lipid is inserted in LNPs comprising methoxy-terminated PEGylated 1,2-diacyl-glycero-lipid.
33. The lipid nanoparticle construct of embodiment 32, wherein the nanoparticle construct is obtainable from incorporating PEGylated 1,2-diacyl-glycero-lipid that has been coupled to the targeting agent via reaction of DBCO-functionalised PEGylated 1,2-diacyl-glycero-lipid with an azide group group at the N-terminus of the targeting agent.
34. The lipid nanoparticle construct of embodiment 32, wherein the nanoparticle construct is obtainable from incorporating PEGylated 1,2-diacyl-glycero-lipid that has been coupled to the targeting agent via reaction of azide-functionalised PEGylated 1,2-diacyl-glycero-lipid with an DBCO-functionalized targeting agent, or
35. The lipid nanoparticle construct of embodiment 32, wherein the nanoparticle construct is obtainable from incorporating PEGylated 1,2-diacyl-glycero-lipid that has been coupled to the targeting agent via reaction of NHS (N-Hydroxysuccinimid)-ester-functionalised PEGylated 1,2-diacyl-glycero-lipid with the amine group at the N-terminus of the targeting agent.
36. The lipid nanoparticle construct of any of the preceding embodiments, further comprising an active agent selected from an RNA, a DNA, a drug, an enzyme or a dye.
37. The lipid nanoparticle construct of embodiment 36, wherein the active agent is an RNA.
38. The lipid nanoparticle construct of embodiment 36, wherein the active agent is a DNA.
39. The lipid nanoparticle construct of any of embodiments 37-38, wherein the active agent encodes a recombinase.
40. The lipid nanoparticle construct of embodiment 39, wherein the recombinase is Brec1.
41. The lipid nanoparticle construct of embodiment 39, wherein the recombinase is a HTLV recombinase.
42. The lipid nanoparticle construct of any of preceding embodiments, wherein the targeting agent is a single domain antibody to human CD4 and wherein the lipid nanoparticle comprises an RNA encoding a recombinase, optionally, Brec1.
43. Use of the lipid nanoparticle construct of any of the preceding embodiments for targeting a cell, e.g., in vitro.
44. Use of embodiment 43, wherein the cell is a T cell.
45. Use of embodiment 43, wherein the cell is a CD4+ T cell.
46. A method of preparing a cell comprising an active agent, comprising contacting said cell with the lipid nanoparticle construct of any of embodiments 1-42 comprising an active agent, e.g., in vitro.
47. The method of claim 46, wherein the active agent is an mRNA encoding a therapeutic protein, and the cell expresses the therapeutic protein.
48. A pharmaceutical composition comprising the lipid nanoparticle construct of any of embodiments 1-42 and a pharmaceutically acceptable solvent.
49. The pharmaceutical composition of embodiment 48 for use in targeting a cell.
50. The pharmaceutical composition for use of embodiment 49, wherein the cell is a T-cell, optionally, a CD4+ T cell.
51. A method of treating a subject in need thereof, comprising administering to the subject a pharmaceutical composition of any of embodiments 48-50, wherein optionally, the subject has a disease or disorder selected from the group comprising an HIV infection, a HTLV infection, cancer or an enzyme deficiency.
52. The method of embodiment 51, wherein the composition is administered systemically.
53. The method of embodiment 51, wherein the composition is administered locally.
54. The method of embodiment 51, wherein the composition is administered intravenously.
55. The method of embodiment 51, wherein the composition is administered intrathecally.
56. The method of embodiment 51, wherein the composition is administered intrapulmonarily.
57. The method of embodiment 51, wherein the composition is administered intramuscularly.
58. The method of embodiment 51, wherein the composition is administered intranasally.
59. The method of embodiment 51, wherein the composition is administered topically, e.g., subcutaneously.
60. The method of embodiment 51, wherein the composition is administered intratumorally.
61. The method of embodiment 51, wherein the composition is administered orally.
62. The method of embodiment 51, wherein the composition is administered to cells, e.g., T cells, ex vivo.
63. A composition or kit suitable for preparing the lipid nanoparticle construct of any of embodiments 1-42 comprising
   a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide, preferably, β-(L-Arginyl)-L-2,3-diamino propionic acid-N-palmityl-N-oleyl-amide, and
   b) CHEMS,
   c) cholesterol, and
   d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group comprising stearoyl, myristoyl, palmitoyl, and oleoyl, preferably, stearoyl,
      wherein said PEGylated 1,2-diacyl-glycero-lipid is selected from maleimide-modified PEGylated 1,2-diacyl-glycero-lipid, DBCO-modified PEGylated 1,2-diacyl-glycero-lipid, azide-modified PEGylated 1,2-diacyl-glycero-lipid, targeting agent linked to the distal PEG-chain end of PEGylated 1,2-diacyl-glycero-lipid and/or methoxy-terminated PEGylated 1,2-diacyl-glycero-lipid
   e) wherein, if the PEGylated 1,2-diacyl-glycero-lipid of d) consists of methoxy-terminated PEGylated 1,2-diacyl-glycero-lipid, the composition or kit further comprises maleimide-modified PEGylated 1,2-diacyl-glycero-lipid, DBCO-functionalised PEGylated 1,2-diacyl-glycero-lipid, azide-functionalised PEGylated 1,2-diacyl-glycero-lipid or targeting agent linked to the distal PEG-chain end of PEGylated 1,2-diacyl-glycero-lipid,
   f) wherein, if no linked targeting agent is present, the kit further comprises the targeting agent in separate form and
   g) optionally, active agent.
64. A kit suitable for preparing the lipid nanoparticle construct of any of embodiments 1-42 comprising
   a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide, and
   b) CHEMS,
   c) cholesterol, and
   d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group comprising stearoyl, myristoyl, palmitoyl, and oleoyl, preferably, stearoyl,
      wherein said PEGylated 1,2-diacyl-glycero-lipid is selected from maleimide-modified PEGylated 1,2-diacyl-glycero-lipid, DBCO-modified PEGylated 1,2-diacyl-glycero-lipid, azide-modified PEGylated 1,2-diacyl-glycero-lipid, targeting agent linked to the distal PEG-chain end of PEGylated 1,2-diacyl-glycero-lipid and/or methoxy-terminated PEGylated 1,2-diacyl-glycero-lipid
   e) wherein, if the PEGylated 1,2-diacyl-glycero-lipid of d) consists of methoxy-terminated PEGylated 1,2-diacyl-glycero-lipid, the kit further comprises maleimide-modified PEGylated 1,2-diacyl-glycero-lipid, DBCO-functionalised PEGylated 1,2-diacyl-glycero-lipid, azide-functionalised PEGylated 1,2-diacyl-glycero-lipidor targeting agent linked to the distal PEG-chain end of PEGylated 1,2-diacyl-glycero-lipid,
   f) wherein, if no linked targeting agent is present, the kit further comprises the targeting agent in separate form and
   g) optionally, active agent.
65. The composition or kit of any of embodiments 63 or 64, comprising LNP comprising components a)-d).
66. A method of preparing the LNP constructs of any of claims 1-42, comprising generating LNP from at least components a)-d) of the kit or composition of any of embodiments 63-65 in a single step process.
67. The method of embodiment 66, comprising a separate coupling step linking the targeting agent to activated PEGylated 1,2-diacyl-glycero-lipid in the LNPs.
68. The method of embodiment 66, comprising a separate step incorporating targeting agent linked to activated PEGylated 1,2-diacyl-glycero-lipid into LNPs comprising methoxy-terminated PEGylated 1,2-diacyl-glycero-lipid.
69. The method of embodiment 66, comprising a separate coupling step linking a streptavidin- or avidin-modified targeting agent to biotinylated PEGylated 1,2-diacyl-glycero-lipid in the LNPs.

### Figures

Fig. 1 Composition of preferred LNP of the invention
Fig. 2 Different methods of producing LNP constructs of the invention A: Coupling of targeting agent to LNP B: Post-insertion of targeting agent linked to PEGylated lipids

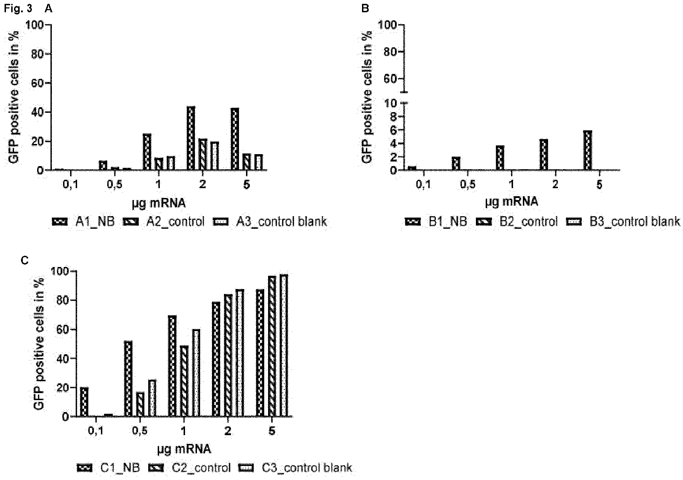

Fig. 3 Frequency of GFP positive cells after transfection with LNPs. Cells of the cell line SupT1 were transfected with three different formulations of LNPs (A/B/C) and respective control constructs at different GFP mRNA concentrations (0,1 - 5 µg). Cells were incubated with formulations for 24 h before analysis via flow cytometry. Flow cytometry was done on a Miltenyi Biotec MACSQuant Analyzer 10.

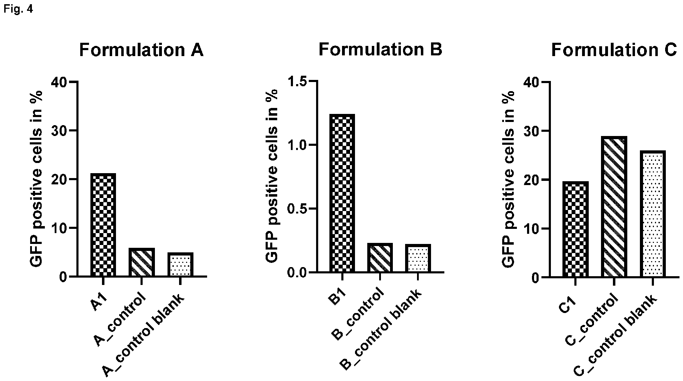

Fig. 4 Frequency of GFP positive cells after transfection with LNPs. Isolated CD4+ T cells were activated for24 h and then transfected with three different formulations of LNPs (A/B/C) and respective control constructs with 5 µg GFP mRNA each. Analysis via flow cytometry 24 h post transfection using a MACSQuant Analyzer 10.

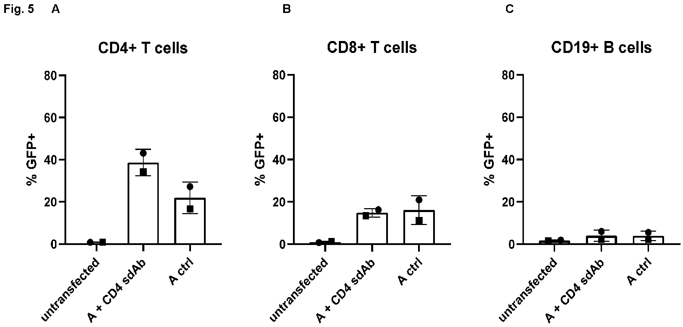

Fig. 5 Frequency of GFP+ CD4+, CD8+ and CD19+ cells after transfection with LNPs. PBMC were activated for 24 h and then transfected with Formulation A and respective control constructs with 20 µg (constructs A) GFP mRNA. Analysis via flow cytometry 24 h post transfection using a MACSQuant Analyzer 10.

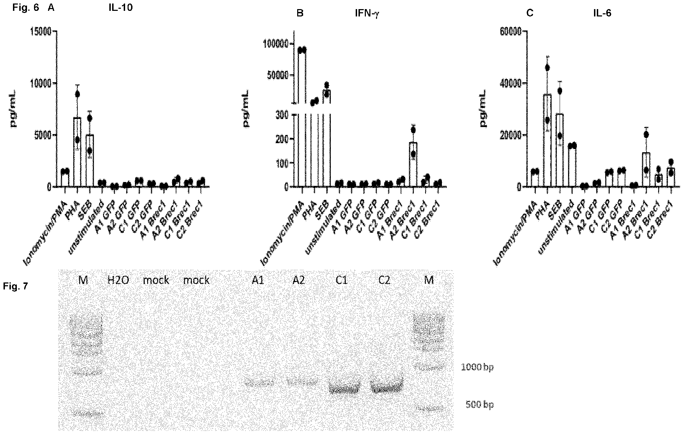

Fig. 6 Comparison of cytokine secretion (A: IL-10, B: IL-6, C: IFN-y) stimulated by LNP constructs. PBMC were transfected with LNPs for 24 hours with different mRNA cargo (GFP or Brec1). A1/C1: LNP without targeting agent/control, A2/C2: LNP with CD4 sdAb as targeting agent. Ionomycin/PMA, PHA or SEB were used as positive control, and "unstimulated" means the absence of any control agent or LNP.
Fig. 7 Agarose gel with PCR amplified genomic DNA of cells transfected with LNPs with primers specific for genomic scar. Mock: Untransfected control cell lines (D1 und D4), H₂O: negative control. A1/A2/C1/C2: Reporter cell line transfected with different constructs. M: size marker. Isolation of genomic DNA after 48 h and end-point PCR with scar specific primers; expected length of fragment is 803 base pairs (bp).

### Examples

### Example 1: Formulation of GFP mRNA in CD4 T-cell targeting lipid nanoparticles LNP105 for in vitrolvivo applications by MALEIMIDE COUPLING - Pre-PEGylation (Method A)

### 1.1 First process step - formulation of Maleimide-functionalized mRNA -LNP

In a first process step, for formulating **LNP105**, the cationic lipid β-(L-Arginyl)-L-2,3-diamino propionic acid-N-palmityl-N-oleyl-amide (PANTH02), 3β-Hydroxy-5-cholestene 3-hemisuccinate (Cholesteryl hemisuccinate, CHEMS), Cholesterol, and the PEGylated lipid 1,2-distearoyl-sn-glycero-3-phosphoethanolamine-N-[maleimide (polyethylene glycol)-2000] (ammonium salt) (DSPE-PEG2k-Maleimide) were dissolved in pure ethanol in a molar ratio of 30 : 49 : 18.5 : 2.5, respectively.

After the first step of formulating the respective lipid solutions, each lipid solution was mixed with CleanCap^{®} GFP mRNA (5moU (=5-methoxy-uridine modification; Trilink Biotechnologies, San Diego), dissolved in 50 mM sodium acetate buffer of pH 4.0, using a microfluidic mixer (NanoAssemblr^{®} Spark^{™}; Precision Nanosystems, Vancouver, Canada) in a fixed mass ratio of 20 : 1 (m[lipid] : m[mRNA]). The aqueous mRNA solution and the ethanolic lipid mixture were combined at a volume ratio of 2:1 (V[aqueous phase] : V[organic phase]) and an overall flow rate of 18 mL/min. The concentration of the mRNA and lipid solutions is adjusted in a way so that a final mRNA concentration in the range of 50-200 µg/mL is obtained after mixing.

After the mixing process, the obtained lipid nanoparticles (LNPs) were transferred to Slide-A-Lyzer dialysis cassettes (MWCO 3.5 kDa, Thermo Scientific, Rockford, IL, USA) and dialyzed overnight against 10 mM Tris (pH 7.5) buffered 270 mM Sucrose solution at 4 °C. During the dialysis process, a total number of three buffer exchange cycles was performed with an external medium volume of at least 500x times the volume of the sample. Instead of sucrose, other sugars like trehalose or glucose can be equally selected for tonicity adjustment and/or as cryo-preservative buffer constituents.

Subsequently, the thus obtained formulations were tested for particle size and ζ-potential surface charge (Zetasizer Ultra instrument (Malvern Instruments Ltd, Malvern, UK), mRNA encapsulation efficiency and total mRNA content (Quant-iT RiboGreen RNA Assay Kit, Thermo Fisher Scientific, Waltham, USA), as well as analyzed for their Osmolality levels (Gonotec Osmomat 3000 Freezing Point Osmometer, ELITechGroup).

All LNP105 particles were found to obtain a Z-Average hydrodynamic diameter of 50 to 90 nm, PDI < 0.15 and ζ-potential of -10.0 mV- +5 mV. The formulations displayed greater than 90 % mRNA encapsulation efficiency and osmolality levels of 250 - 320 mOsmol/kg.

### 1.2a Second process step - coupling of a Nanobody via thio-Michael addition

In a second process step, an aqueous solution of the pre-reduced CD4-targeting Nanobody (CD4-Nb1, 15.57 kDa, 145 Amino acids, NMI Naturwissenschaftliches und Medizinisches Institut an der Universität Tübingen, Reutlingen, Germany; WO 2023/275075 A1) was directly mixed with the formulated maleimide - surface bearing LNPs at a fixed molar ratio of 1:200 or 1:250 of CD4-NB : DSPE-PEG2k-Maleimide lipid, and the mixture reacted under mild agitation at pH 7.5 overnight at 4 °C. The conjugation is based on the thio-Michael addition reaction, where the sulfhydryl group of the cysteine residue of the C-terminus site of the CD4-NB nucleophilically reacts with the α,β-unsaturated Michael acceptor i.e., the maleimide group of the DSPE-PEG2k-Maleimide.

After the mixing process, the now CD4-NB functionalized LNP formulations were transferred to Spectra-Por^{®} Float-A-Lyzer^{®} G2 dialysis tubes (100-300 kDa MWCO, CE, 1 mL, Spectrum Lab / Repligen, Waltham, USA) and dialyzed overnight against 10 mM Tris (pH 7.5) buffered 270 mM Sucrose solution at 4 °C for the removal of unreacted Nanobody species. During the purification process, a total number of three buffer exchange cycles was performed with an external medium volume of at least 500x times the volume of the sample.

CD4-NB-coupled LNP105 were found to obtain a Z-Average hydrodynamic diameter of 50 to 90 nm, PDI < 0.15 and ζ-potential of -10.0 mV - +5 mV. The formulations displayed greater than 90 % mRNA encapsulation efficiency and osmolality of 250 - 320 mOsmol/kg.

The obtained mRNA-LNP formulations were stored at -80 °C until further use.

### 1.2b Second process step - coupling of N-Acetyl Cysteine via thio-Michael addition, negative control LNPs which are not bearing Single domain antibodies

In an alternative second process step, a 0.1 M aqueous solution of N-Acetyl Cysteine (Thermo Scientific, Waltham, USA) was directly mixed with the formulated maleimide - surface bearing LNPs at a fixed molar ratio of 1: 500 of DSPE-PEG2k-Maleimide lipid : Cysteine, and the mixture reacted under mild agitation at pH 7.5 overnight at 4 °C. The conjugation is based on the thio-Michael addition reaction, where the sulfhydryl group of the cysteine nucleophilically reacts with the α,β-unsaturated Michael acceptor i.e., the maleimide group of the DSPE-PEG2k-Maleimide.

After the mixing process, the now cysteine functionalized LNP formulations were transferred to Spectra-Por^{®} Float-A-Lyzer^{®} G2 dialysis tubes (100-300 kDa MWCO, CE, 1 mL, Spectrum Lab / Repligen, Waltham, USA) and dialyzed overnight against 10 mM Tris (pH 7.5) buffered 270 mM Sucrose solution at 4 °C for the removal of unreacted cysteine. During the purification process, a total number of three buffer exchange cycles was performed with an external medium volume of at least 500x times the volume of the sample.

N-Acetyl Cysteine-coupled LNP105 were found to obtain a Z-Average hydrodynamic diameter of 50 to 90 nm, PDI < 0.15 and ζ-potential of -10.0 mV - +5 mV. The formulations displayed greater than 90 % mRNA encapsulation efficiency and osmolality of 250 - 320 mOsmol/kg.

The obtained mRNA-LNP formulations were stored at -80 °C until further use.

### Example 2 : Formulation of GFP mRNA in CD4 T-cell targeting lipid nanoparticles LNP105 (formulation A) for in vitro/vivo applications by NHS COUPLING - Post-PEGylation (Method B)

### 2.1 First process step - formulation of PEG-NHS mRNA-LNPs

In a first process step, for formulation of LNP105, the cationic lipid β-(L-Arginyl)-L-2,3-diamino propionic acid-N-palmityl-N-oleyl-amide, 3β-Hydroxy-5-cholestene 3-hemisuccinate (Cholesteryl hemisuccinate, CHEMS), Cholesterol, and the PEGylated lipid 1,2-dimyristoyl-sn-glycero-3-phosphoethanolamine-N-[methoxy(polyethylene glycol)-2000] (ammonium salt) was dissolved in pure ethanol in a molar ratio of 29 : 49 : 19.5 : 2.5, respectively.

In a second process step, the respective lipid solution is mixed with CleanCap^{®} GFP mRNA (5moU (=5-methoxy-uridine modification; Trilink Biotechnologies, San Diego), dissolved in 50 mM sodium acetate buffer of pH 4.0, using a microfluidic mixer (NanoAssemblr^{®} Spark^{™}; Precision Nanosystems, Vancouver, Canada) in a fixed mass ratio of 20 : 1 (m[lipid] : m[mRNA]). The aqueous mRNA solution and the ethanolic lipid mixture are combined at a volume ratio of 2:1 (V[aqueous phase] : V[organic phase]) and an overall flow rate of 18 mL/min. The concentration of the mRNA and lipid solutions is adjusted in a way so that a final mRNA concentration in the range of 50-200 µg/mL is obtained after mixing.

After the mixing process, the obtained lipid nanoparticles (LNPs) are transferred to Slide-A-Lyzer dialysis cassettes (MWCO 3.5 kDa, Thermo Scientific, Rockford, IL, USA) and dialyzed overnight against 10 mM Tris (pH 7.5) buffered 270 mM Sucrose solution at 4 °C. During the dialysis process, a total number of three buffer exchange cycles was performed with an external medium volume of at least 500x times the volume of the sample. Instead of sucrose, other sugars like trehalose or glucose can be equally selected for tonicity adjustment and/or as cryo-preservative buffer constituents.

Subsequently, the thus obtained formulations are tested for particle size and ζ-potential surface charge (Zetasizer Ultra instrument (Malvern Instruments Ltd, Malvern, UK), mRNA encapsulation efficiency and total mRNA content (Quant-iT RiboGreen RNA Assay Kit, Thermo Fisher Scientific, Waltham, USA), as well as analyzed for their Osmolality levels (Gonotec Osmomat 3000 Freezing Point Osmometer, ELITechGroup).

### 2.2 Second process step - coupling of a Nanobody via Amino - NHS coupling reaction

The CD4-targeting Nanobody Nb1 is coupled to DSPE-PEG2k-NHS via amino-NHS coupling reaction.

DSPE-PEG2k-NHS was dissolved in a 2 : 1 (V/V) mixture of 200 mM Sodium Bicarbonate (pH 8.7) and Ethanol at a final concentration of 6 mg/ml. A

An appropriate amount of the DSPE-PEG2k-NHS solution was afterwards mixed at a defined molar ratio (e.g., in a range of 50 to 200 : 1 mol/mol) with the CD4-targeting Nanobody Nb1 which was predissolved in 20 mM Phosphate buffer pH 6.8. The reaction mixture was stirred over night at 4 °C. After this coupling process, the reaction mixture was transferred to Spectra-Por^{®} Float-A-Lyzer^{®} G2 dialysis tubes (100-300 kDa MWCO, CE, 1 mL, Spectrum Lab / Repligen, Waltham, USA) and dialyzed overnight against 10 mM Tris (pH 7.5) buffered aqueous solution at 4 °C. During the dialysis process, a total number of three buffer exchange cycles was performed with an external medium volume of at least 500x times the volume of the sample.

### 2.3 Third process step - Post-insertion of NB-coupled NHS micelles to mRNA-LNPs

Current models indicate that post-insertion of PEG species into LNPs occurs via the interaction of micellar intermediate forms from which monomer PEG-lipid chains are released and thereafter preferentially incorporated onto the outer surface of LNPs.

Therefore, an aqueous solution of CD4-targeting Nanobody coupled PEG2k-NHS micelles is directly mixed with the pre-formed LNP formulation at predetermined molar ratios of 0.5, 0.75, 1.0 and 1.5 % (PEG-NHS micelles : LNPs), and the mixture left to incubate at 40 °C for 1.5 hour under vigorous stirring (1000 rpm, Eppendorf Thermomixer C, Germany). After the mixing process, the now functionalized LNP formulations are transferred to Slide-A-Lyzer^{™} MINI Dialysis Devices (10kDa MWCO, Thermo Scientific, Rockford, IL, USA) and dialyzed overnight against 10 mM Tris (pH 7.5) buffered 270 mM Sucrose solution at 4 °C for the removal of unreacted species. During the purification process, a total number of three buffer exchange cycles is performed with an external medium volume of at least 28x times the volume of the sample.

CD4-targeting Nanobody coupled LNPs were found to obtain a Z-Average hydrodynamic diameter of 90 to 120 nm, PDI < 0.2 and ζ-potential of -10 mV +/- 10 mV. The formulations displayed greater than 90 % mRNA encapsulation efficiency and osmolality of 250 - 320 mOsmol/kg.

The obtained mRNA-LNP formulations are stored at -80 °C until further use.

### Example 3: Formulation of GFP mRNA in CD4 T-cell targeting lipid nanoparticles for in vitrolvivo applications by Azide/DBCO COUPLING - Pre-PEGylation (Method C)

### 3.1 First process step - formulation of Azide-functionalized mRNA-LNP

In a first process step, for preparation of LNP105, the cationic lipid β-(L-Arginyl)-L-2,3-diamino propionic acid-N-palmityl-N-oleyl-amide, 3β-Hydroxy-5-cholestene 3-hemisuccinate (Cholesteryl hemisuccinate, CHEMS), Cholesterol, and the PEGylated lipid 1,2-distearoyl-sn-glycero-3-phosphoethanolamine-N-[dibenzocyclooctyne(polyethylene glycol)-2000] (ammonium salt) (DSPE-PEG2k-DBCO) are dissolved in pure ethanol in a molar ratio of 30 : 49 : 18.5 : 2.5, respectively.

Subsequently, the lipid solution is mixed with CleanCap^{®} GFP mRNA (5moU (=5-methoxy-uridine modification; Trilink Biotechnologies, San Diego), dissolved in 50 mM sodium acetate buffer of pH 4.0, using a microfluidic mixer (NanoAssemblr^{®} Spark^{™}; Precision Nanosystems, Vancouver, Canada) in a fixed mass ratio of 20 : 1 (m[lipid] : m[mRNA]). The aqueous mRNA solution and the ethanolic lipid mixture were combined at a volume ratio of 2:1 (V[aqueous phase] : V[organic phase]) and an overall flow rate of 18 mL/min. The concentration of the mRNA and lipid solutions is adjusted in a way so that a final mRNA concentration in the range of 50-200 µg/mL is obtained after mixing.

After the mixing process, the obtained lipid nanoparticles (LNPs) are transferred to Slide-A-Lyzer dialysis cassettes (MWCO 3.5 kDa, Thermo Scientific, Rockford, IL, USA) and dialyzed overnight against 270 mM Sucrose in 10 mM Tris (pH 7.5) buffer at 4 °C. During the dialysis process, a total number of three buffer exchange cycles is performed with an external medium volume of at least 500x times the volume of the sample. Instead of sucrose, other sugars like trehalose or glucose can be equally selected for tonicity adjustment and/or as cryo-preservative buffer constituents.

Subsequently, the thus obtained formulations are tested for particle size and ζ-potential surface charge (Zetasizer Ultra instrument (Malvern Instruments Ltd, Malvern, UK), mRNA encapsulation efficiency and total mRNA content (Quant-iT RiboGreen RNA Assay Kit, Thermo Fisher Scientific, Waltham, USA), as well as analyzed for their Osmolality levels (Gonotec Osmomat 3000 Freezing Point Osmometer, ELITechGroup).

### 3.2 Second process step - coupling of a Nanobody via Azide - DBCO click reaction

In a second process step, an aqueous solution of the Azide-modified CD4-targeting Nanobody CD4-targeting Nanobody (CD4-Nb1 variant, NMI Naturwissenschaftliches und Medizinisches Institut an der Universität Tübingen, Reutlingen, Germany) is directly mixed with the formulated DBCO - surface bearing LNPs at a fixed molar ratio of 1:200 or 1:250 of CD4-NB : DSPE-PEG2k-DBCO lipid, and the mixture reacted under mild agitation at pH 7.5 overnight at 4 °C. The conjugation is based on the strain promoted alkyne azide cycloaddition (SPAAC) where the azide groups exclusively react with the DBCO-tagged PEG-lipids to form a stable triazole.

After the mixing process, the nowCD4-NB functionalized LNP formulations are transferred to Spectra-Por^{®} Float-A-Lyzer^{®} G2 dialysis tubes (100-300 kDa MWCO, CE, 1 ml, Spectrum Lab / Repligen, Waltham, USA) and dialyzed overnight against 10 mM Tris (pH 7.5) buffered 270 mM Sucrose solution at 4 °C forthe removal of unreacted Nanobody species. During the purification process, a total number of three buffer exchange cycles is performed with an external medium volume of at least 500x times the volume of the sample.

The obtained mRNA-LNP formulations are stored at -80 °C until further use.

As controls for the antibody-linked LNP constructs generated via click-reactions, the LNP not reacted with ligand can be used, which carry DBCO or azide on their surface, which can only react with azide or DBCO, accordingly, and do not react with cellular components like thiol-reactive maleimides. Alternatively, non-target related antibodies, e.g., non-target related single domain antibodies can be used for control.

### Example 4:

Lipid nanoparticles LNP105 (Formulation A) as prepared by the method of Example 1, and comparative formulations LNP107 (Formulation B) and LNP133 (Formulation C) comprising different lipids were used with the goal of specifically transfecting cells expressing a defined cell surface protein, in particular, CD4+ T cells. The specificity of the LNPs is conveyed by a sdAb on the surface of the LNPs.

CD4+ T cells are the main target cells of the Human Immunodeficiency Virus (HIV). The LNPs carry messenger RNA (mRNA) encoding for the Brec1 recombinase (WO2016/034553A1). The sdAb coupled to the surface of the LNPs (CD4-Nb1, NMI Naturwissenschaftliches und Medizinisches Institut an der Universität Tübingen, Reutlingen, Germany; WO 2023/275075 A1) is expected to mediate binding to CD4 on T cells. After binding to this receptor, the LNPs are either taken up by the cell via endocytosis or merge with the cell membrane. In both cases, the mRNA encoding for the Brec1 is translocated into the cytosol, where the protein is made by the cell machinery. If the transfected cell is infected with HIV, the recombinase can specifically recognize the proviral genome and cut it out from the host cell genome. The excision product will be broken down by the cell, leading to a "cured" cell free from HIV. There is no genetic modification of the host cell because only the protein is produced and degraded after a short amount of time.

The first experiments were conducted with a surrogate mRNA instead of mRNA encoding for Brec1. The mRNA used leads to the production of the marker green fluorescent protein (GFP), which is easily detectable by flow cytometry. Also, cell lines instead of human primary cells were used in some experiments. Cell lines are easier to maintain and to transfect than primary cells.

Briefly, CD4+ T cells isolated from human PBMC (e.g., by negative selection from buffy coats, for example using EasySep^{™} Human CD4+ T Cell Isolation Kit (Stemcell Technologies, #17952RF) were activated for 24 h by incubation with CD3/CD28 dynabeads (Gibco, #1113D) before different amounts of LNPs and respective control particles were added. Cell lines were transfected without prior activation. The mixture was then incubated for another 24 hours before the frequency of GFP positive cells was analysed via flow cytometry.

For the first experiments, the T-cell line SupT1 was used. These cells highly express CD4 on their surface. The results are shown in Figure 3. For each different LNP construct, LNP comprising the sdAb coupled to the surface were compared with control samples of the respective LNP. The difference between the two control samples, i.e., Blank LNPs and the N-Acetyl Cysteine-coupled LNPs is basically that the latter ones are covered/'masked' with the blocking agent N-Acetyl cysteine. More specifically, in the first case, the Blank LNPs bear the free thiol-reactive maleimide groups on their surface. In the second case, for the control, these free maleimide groups are blocked with the reactive thiol-bearing N-Acetyl Cysteine molecule. For the LNP construct of the invention, the maleimide groups are reacted with the sdAb (nanobody) species.

Formulations A and C yielded a substantial amount of GFP positive cells after transfection with 5 µg or less of GFP mRNA. In the case of formulation A, the transfection was dependent on the sdAb: 40 % of cells were expressing GFP after transfection with the A1_NB, while only approximately 10 % of the cells were GFP+ using A2_control or A3_control blank.

Formulation C transfected the SupT1 independently of the sdAb. Almost 100 % of the cells were GFP+ after adding 5 µg mRNA with C2_control or C2_control blank, achieving higher values than C1_NB.

The frequency of GFP+ cells after transfection with formulation B was very low, i.e. less than 10 %, regardless of the presence or absence of the sdAb.

After the first experiments in a cell line, human primary cells were used. In a first step, isolated CD4+ T cells were transfected. Results are shown in Figure 4.

In isolated primary CD4+ T cells, the frequency of GFP positive cells after transfection was similar between formulation A and C (approx. 20 % for constructs with sdAb). Again, formulation A was sdAb dependent, while formulation C was independent and achieved higher frequencies of transfected cells in the controls. Similar to the experiments conducted with SupT1 cells, the transfection with formulation B resulted in a very low frequency of GFP+ cells (less than 2 %), regardless of the sdAb.

In the subsequent experiments, peripheral blood mononuclear cells (PBMC) were used for transfection. This was done to assess the specificity of the different LNP constructs in a mixture of different cell types. Figure 5 shows the frequencies of GFP+ CD4+, CD8+ and CD19+ cells after transfection.

After transfection of PBMC with LNPs, an sdAb dependent effect was observed for formulation A in CD4+ T cells and to a lesser extent in CD8+ T cells. The construct A1 resulted in approx. 25 % GFP+ CD4+ T cells and approx. 15 % GFP+ CD8+ T cells. Transfection with formulation C led to higher frequencies of GFP+ CD4+ than formulation A (approx. 40 % GFP+ cells), but also a higher background in CD8+ T cells. There, the control and the control blank construct led to even higher frequencies of GFP+ cells (25-30%) than the construct with sdAb. B cells were not transfected efficiently by any of the constructs, i.e. the frequency of GFP+ B cells was very low.

To assess a potential harmful immune reaction by the secretion of inflammatory cytokines such as interleukin 6 (IL-6) or interferon gamma (IFN-y), PBMC were transfected with LNP for 24 hours with different mRNA cargo (GFP or Brec1). Supernatants were collected and analyzed for secreted cytokines. Chemicals that induce a strong immune reaction (lonomycin, PMA, PHA and SEB) were used as positive controls while unstimulated, untransfected cells served as control for basal cytokine expression. Results are shown in Fig. 6.

In comparison to the positive controls and to unstimulated, untransfected cells, PBMC transfected with formulation A secreted lower or comparable levels of IL-6, IL-10, IL-17, TNF-a and IFN-γ as well as other cytokines (representative results for IL-6, IL10 and IFN-γ shown in Fig. 6). Only transfection with LNP constructs of the invention carrying Brec1 mRNA yielded a higher concentration of some cytokines, e.g., IL-6 or IFN-γ in the supernatant compared to untransfected cells. However, this was still considerably lower than the positive controls. Thus, the LNP constructs of the invention do not per se activate T cells to secrete pro-inflammatory or anti-inflammatory cytokines, which is an important safety characteristic for administration to a human subject. In comparison, the LNP constructs of formulation C led to a significantly higher expression of some cytokines with or without expression of BREC1, although still less than the positive controls.

After assessing the transfection efficacy with a surrogate mRNA encoding for the GFP, it was tested whether the recombinase Brec1 could be functionally expressed in cells transfected with LNPs. To this end, a cell line was used that expresses CD4 on its surface and has a full HIV genome integrated but is not replication competent (SupT1 clone A3). After successful recognition and recombination of the HI provirus by the recombinase, a circular excision product can be amplified by polymerase chain reaction (PCR) (see schematic picture x, part II).

Also, a so called "genomic scar" can be amplified via PCR. The "scar" is a part of the long-terminal-repeats sequence of the virus which remains in the host cell genome after recombination. Results of one experiment are shown in Figure 7. A band at around 800 base pairs (bp) is visible in the samples that were transfected with A1, A2, C1 and C2. Since the expected length of the scar fragment is 803 bp, it is assumed that the Brec1 can be functionally expressed after transfection of mRNA via LNPs. No sdAb dependent effect could be observed.

In summary, the experiments conducted so far demonstrate an sdAb-dependent effect of formulation A and the feasibility of efficiently and specifically transfecting primary human CD4 T cells with GFP mRNA. Formulation C is less specific but yields to high rates of transfected cells.

Furthermore, mRNA transfection with formulations A and C leads to the functional expression of Brec1 in a reporter T cell line which expresses CD4 on its surface.

## Claims

1. A lipid nanoparticle construct comprising a hydrophilic lipid nanoparticle comprising
a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide,
b) 3β-Hydroxy-5-cholestene 3-hemisuccinate (CHEMS),
c) cholesterol, and
d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group consisting of stearoyl, myristoyl, palmitoyl, and oleoyl,
and a targeting agent linked to the distal PEG-chain end of at least a part of the PEGylated 1,2-diacyl-glycero-lipid.

2. The lipid nanoparticle construct of claim 1, wherein the lipid nanoparticle further comprises a methoxy-terminated PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group consisting of stearoyl, myristoyl, palmitoyl, and oleoyl.

3. The lipid nanoparticle construct of any of the preceding claims, wherein the cationic lipid is β- (L-Arginyl)-L-2,3-diamino propionic acid-N-palmityl-N-oleyl-amide.

4. The lipid nanoparticle construct of any of the preceding claims, wherein the PEGylated 1,2-diacyl-glycero-lipid is 1,2-diacyl-glycero-3-phosphoethanolamine-N-polyethylene glycol, preferably, 1,2-distearoyl-sn-glycero-3-phosphoethanolamine-N-polyethylene glycol (DSPE-PEG), optionally, DSPE-PEG2000.

5. The lipid nanoparticle construct of any of the preceding claims, wherein the molar ratio of the lipids in the lipid nanoparticle is
from 20-40 mol% cationic lipid, preferably, β-(L-Arginyl)-L-2,3-diamino propionic acid-N-palmityl-N-oleyl-amide
from 39-59 mol% CHEMS
from 8.5-28.5 mol% cholesterol and
from 0.5-5 mol% PEGylated 1,2-diacyl-glycero-lipid, preferably, DSPE-PEG,
wherein the overall lipid content is 100%,
wherein optionally the molar ratio cationic lipid : CHEMS : cholesterol: PEGylated 1,2-diacyl-glycero-lipid is 30:49:18.5:2.5.

6. The lipid nanoparticle construct of any of the preceding claims, wherein the targeting agent is capable of binding to a surface antigen of a hematopoietic cell, preferably, a T cell, optionally, a CD4+ T cell.

7. The lipid nanoparticle construct of any of the preceding claims, wherein the targeting agent is selected from the group consisting of an antibody, an antigen binding fragment of an antibody and a ligand to a cell-surface receptor and a fragment of said ligand capable of binding to said receptor,
preferably, wherein the targeting agent is a single domain antibody.

8. The lipid nanoparticle construct of any of the preceding claims, wherein the targeting agent is an antibody or an antigen binding fragment thereof capable of binding to CD4, optionally, to human CD4.

9. The lipid nanoparticle construct of any of the preceding claims, obtainable from
(a) coupling reduced targeting agent to maleimide-modified PEGylated 1,2-diacyl-glycero-lipid that has been incorporated in an LNP or
(b) coupling azide-modified targeting agent to dibenzocyclooctyne (DBCO)-modified PEGylated 1,2-diacyl-glycero-lipid that has been incorporated in an LNP, or
(c) coupling dibenzocyclooctyne (DBCO)-modified targeting agent to azide-modified PEGylated 1,2-diacyl-glycero-lipid that has been incorporated in an LNP.

10. The lipid nanoparticle construct of any of claims 1-9, wherein the targeting agent coupled to PEGylated 1,2-diacyl-glycero-lipid is inserted in LNPs comprising methoxy-terminated PEGylated 1,2-diacyl-glycero-lipid,
optionally, wherein
a) the nanoparticle construct is obtainable from incorporating PEGylated 1,2-diacyl-glycero-lipid that has been coupled to the targeting agent via reaction of DBCO-functionalised PEGylated 1,2-diacyl-glycero-lipid with an azide group group at the N-terminus of the targeting agent, or
b) the nanoparticle construct is obtainable from incorporating PEGylated 1,2-diacyl-glycero-lipid that has been coupled to the targeting agent via reaction of azide-functionalised PEGylated 1,2-diacyl-glycero-lipid with an DBCO-functionalized targeting agent, or
c) the nanoparticle construct is obtainable from incorporating PEGylated 1,2-diacyl-glycero-lipid that has been coupled to the targeting agent via reaction of NHS (N-Hydroxysuccinimid)-ester-functionalised PEGylated 1,2-diacyl-glycero-lipid with the amine group at the N-terminus of the targeting agent.

11. The lipid nanoparticle construct of any of the preceding claims, further comprising an active agent selected from an RNA, a DNA, a drug, an enzyme or a dye, preferably, RNA.

12. The lipid nanoparticle construct of claim 11, wherein the active agent is an RNA encoding a recombinase, optionally, Brec1.

13. The lipid nanoparticle construct of any of the preceding claims, wherein the targeting agent is a single domain antibody to human CD4 and wherein the lipid nanoparticle comprises an RNA encoding a recombinase, optionally, Brec1.

14. Use of the lipid nanoparticle construct of any of the preceding claims for in vitro targeting a cell such as a T cell, preferably, a CD4+ T cell.

15. A method of preparing a cell comprising an active agent, comprising contacting said cell with the lipid nanoparticle construct of any of claims 1-13 comprising an active agent, wherein, optionally, the active agent is an mRNA encoding a therapeutic protein, and the cell expresses the therapeutic protein,
wherein the method can be an in vitro method.

16. A pharmaceutical composition comprising the lipid nanoparticle construct of any of claims 1-13 and a pharmaceutically acceptable solvent, preferably, for use in targeting a cell such as a T-cell, optionally, a CD4+ T cell.

17. A composition or kit suitable for preparing the lipid nanoparticle construct of any of claims 1-13 comprising
a) a cationic lipid selected from the group consisting of β-(L-Arginyl)-L-2,3-diamino propionic acid-N-palmityl-N-oleyl-amide and β-(L-Arginyl)-L-β-alanine-N-palmityl-N-oleyl-amide, and
b) CHEMS,
c) cholesterol, and
d) a PEGylated 1,2-diacyl-glycero-lipid selected from the group consisting of 1,2-diacyl-glycero-3-phosphoethanolamine-N-polyethylene glycol and 1,2-diacyl-glycero-3-O-polyethylenglycol, wherein the acyl groups are independently selected from a group comprising stearoyl, myristoyl, palmitoyl, and oleoyl, preferably, stearoyl,
wherein said PEGylated 1,2-diacyl-glycero-lipid is selected from maleimide-modified PEGylated 1,2-diacyl-glycero-lipid, DBCO-modified PEGylated 1,2-diacyl-glycero-lipid, azide-modified PEGylated 1,2-diacyl-glycero-lipid, targeting agent linked to the distal PEG-chain end of PEGylated 1,2-diacyl-glycero-lipid, biotinylated PEGylated 1,2-diacyl-glycero-lipid and/or methoxy-terminated PEGylated 1,2-diacyl-glycero-lipid
e) wherein, if the PEGylated 1,2-diacyl-glycero-lipid of d) consists of methoxy-terminated PEGylated 1,2-diacyl-glycero-lipid, the composition or kit further comprises maleimide-modified PEGylated 1,2-diacyl-glycero-lipid, DBCO-functionalised PEGylated 1,2-diacyl-glycero-lipid, azide-functionalised PEGylated 1,2-diacyl-glycero-lipidor targeting agent linked to the distal PEG-chain end of PEGylated 1,2-diacyl-glycero-lipid,
f) wherein, if no linked targeting agent is present, the kit further comprises the targeting agent in separate form and
g) optionally, active agent.
